# EUROPEAN PATENT SPECIFICATION

(11) **EP 1 242 132 B1**
(45) Date of publication and mention of the grant of the patent: **15.10.2003**
(21) Application number: 00981559.8
(22) Date of filing: 21.12.2000
(51) Int. Cl.: A61M 5/30, A61M 5/20

(54) **INJECTION DEVICE AND PROPULSION SYSTEM THEREFOR**
INJEKTIONSVORRICHTUNG UND ANTRIEBSSYSTEM DAFÜR
DISPOSITIF D'INJECTION ET SYSTEME DE PROPULSION ASSOCIE

(30) Priority: 23.12.1999 EP 99811206; 17.01.2000 EP 00810037; 13.10.2000 EP 00121397
(43) Date of publication of application: 25.09.2002
(73) Proprietor: Neracher, Arnold, CH-1247 Anières (CH)
(72) Inventor: Neracher, Arnold, CH-1247 Anières (CH)
(74) Representative: Reuteler, Raymond Werner
(86) International application number: IB0001949
(87) International publication number: WO01047586

(56) References cited:
- DE-A- 2 254 153
- US-A- 2 687 725
- US-A- 3 784 179
- US-A- 4 342 310
- US-A- 5 209 303

## Description

### Background of the Invention

The present invention relates to a device for injecting liquids, in particular for intracutaneous or subcutaneous injection of medicaments or other pharmaceutical compositions, such as vaccines. The invention also relates to a propulsion system for or of an injection device.

Manually operated syringes with needles are the most common form of hypodermic injection devices. They have the advantage of being reliable and low cost. The disadvantages are, inter alia, the risk of transmitting diseases by re-use of the syringe, and the pain felt by the patient.

In view of these disadvantages, there have been many attempts to provide needleless hypodermic injection devices in which a liquid to be injected is propelled at high speed by a pressure generator, thereby piercing the skin of a human or animal patient. Such devices are, for example, described in patent publications US 3,527,212, US 2,687,725, US 4,596,556, US 4,722,728, US 4,874,367, US 4,966,581, US 5,501,666 and WO 98/41250. In order to ensure sterility and avoid contamination of medicaments to be injected, certain conventional devices as described in patents US 4,874,367 and US 4,966,581 comprise disposable cartridges. The devices described in these patents are very complex and made of a large number of pieces. They are also bulky, costly and limited in their performance, particularly as concerns the injection pressure and jet diameter which are in the order of 70 bars or less and 100 to 330 µm, respectively, although initial peak pressure may attain around 300 bars. Insufficient pressure and a large diameter jet increases pain and the risk that only a portion of the medicament is injected, especially with respect to patients having a resistant skin. The effectiveness of injection is important, particularly with patients such as diabetics who administer injections daily.

Disposable syringes with pressurised gas propulsion systems, for example as described in WO 98/41250, would not only be difficult to manufacture, but would also cause some safety concerns in view of the large expansion of gas in the event of rupture of the system. Such a device would also be very difficult to seal effectively.

Considering the abovementioned disadvantages, an object of the present invention is to provide a hypodermic injection device that is sterile, effective and reliable. It is advantageous to provide a hypodermic injection device that is compact and cost effective. It is advantageous to provide an injection device that is safe to operate. It is advantageous to provide an injection device that eliminates the risk of disease transmission by re-use. It is advantageous to provide an injection device that is painless to use. In certain applications it is advantageous to provide a hypodermic injection device with the aforementioned advantages that is nevertheless adapted for single use.

### Summary of the Invention

Objects of the invention have been achieved by the propulsion system according to claim 1.

Disclosed herein is a propulsion system for an injection device, said propulsion system comprising a container, a source of potential energy for propelling a fluid with sufficient pressure through an orifice to create a jet enabling subcutaneous delivery of the fluid, the source of potential energy primarily being in the form of a compressible substance under pressure within the container, whereby said potential energy is compression energy of said substance, wherein said compressible substance is a liquid, solid or other non-gaseous substance, as defined at ambient temperature and pressure.

Also disclosed herein is a propulsion system suitable for a single use injection device, said propulsion system comprising a container, a source of potential energy for propelling a fluid with sufficient pressure through an orifice to create a jet enabling subcutaneous or intracutaneous delivery of the fluid, wherein the source of potential energy comprises a first compressible substance at a first pressure P1 within the container and at least a second compressible substance at a second pressure P2 lower than P1, whereby said potential energy is substantially compression energy of said substances, said first substance being a liquid, solid, or other non-gaseous substance as defined at ambient temperature and pressure.

Also disclosed herein is a needleless hypodermic injection device for subcutaneous administration of a liquid product to be injected, such as a medicament, a vaccine or other pharmaceutical composition, comprising a container containing the product to be injected and a liquid or solid or other non-gaseous compressible substance, a nozzle portion with an orifice, and retaining means enabling the compressible substance to remain compressed, prior to use, at a pressure sufficient to propel the liquid product through the orifice so as to create a liquid jet with a velocity sufficient to pierce the skin of a patient.

Also disclosed herein is a hypodermic injection device for sub-cutaneous administration of a liquid product to be injected, such as a medicament, a vaccine, or other pharmaceutical compositions, comprising a container and a source of potential energy primarily being in the form of a compressible substance contained under pressure in the container, a movable skin piercing member comprising a nozzle having a liquid outlet orifice, the skin piercing member being adapted to move beyond a front applicator end of the device to pierce the skin of a patient upon actuation of the device by means of pressure exerted by the compressible substance against the skin piercing member.

The compressible substance may, for example, be a soft matter or other visco-elastic substance, such as a substance belonging to the family of polysiloxanes, which is not expensive and has a large elastic compression range. Certain polysiloxanes may be compressed up to 2000 bars with a 15% volume reduction. Polysiloxanes comprise a volumetric compressibility (dV/V) which is in the range of two to four times greater than the volumetric compressibility of water.

In view of the very high pressure and small orifice diameter, it is possible to produce a liquid jet of supersonic speed. Moreover, the injection time may be spread over a few seconds in view of the small jet diameter (e.g. 30-60 µm) thereby reducing or eliminating pain by giving more time for the medicaments to diffuse in the surrounding tissue.

The provision of a compressed liquid or solid as a source of potential energy for propelling a liquid to be injected is very advantageous over prior art systems using mechanical energy sources such as springs, or compressed gas. The use of springs, for example, requires large dimensions to obtain the required propulsion energy and is unsuitable for single use disposable injection devices. Prior systems using compressed gas, as defined at ambient temperature and pressure, are limited by the maximum pressure of the gas until a change of state to the liquid form, which defines the maximum pressure of the propulsion system. For example, carbon dioxide liquefies at approximately 70 bars and nitrogen protoxide at 75 bars, these gases being the most frequently considered for use in conventional propulsion systems. The large volume change of a compressed gas is also a safety concern, since in the event of rupture of the gas container, loose particles of the device are driven by the large expansion of gas liberated from the container.

Preferred compressible substances used in the invention, such as polysiloxane oils or gels, or vulcanised silicon rubber, which may be compressed for example to 2000 bars to obtain up to 15% volume reduction, do not cause an explosion in the event of rupture. Furthermore, a liquid or solid compressible substance can be loaded in a container at much higher pressure since there is no change of state and the substance escapes less easily through the sealing joints than gaseous substances. Vulcanized silicon rubber or high molecular weight polysiloxane oils, for example, which are very viscous, are much easier to contain without leakage through seals compared to gas and even liquids with low viscosity such as water. In conventional gas-propelled systems, where the gas is liquefied, pressures beyond 100 or 200 bars would be extremely difficult to maintain over a length of time required for the shelf life of typical pharmaceutical or medical products since the gas would leak through joints of the propulsion system, for example around the piston seals. While polysiloxane oils or gels are preferred substances in view of the combination of high viscosity, relatively high compressibility and low cost, numerous other substances with compressibility greater than water and preferably greater than double the compressibility of water could be implemented in certain embodiments of the invention. Examples of other compressible substances that may be implemented in the present invention are cork, polyurethane and butyl polymers. These substances have volumetric compressibility ratios (dV/V) in the range 1,2 to 2 times that of water.

In the invention, although the principal source of potential energy stems from the compressed substance, it need not be the unique source. In this regard, the compressible substance may comprise dissolved gas, or a spring may be further provided. The compressed substance liberates energy in an initial phase of high-pressure injection, followed by liberation of energy from the gas or spring at relatively low pressure. In the latter embodiments, the compressed substance would provide an energy source in a compact form in order to produce initial high pressure for the purposes of piercing a patient's skin, the lower pressure energy sources being sufficient to complete injection after the patient's skin has been pierced. The high energy density that may be stored in compressible substances according to this invention enables the hypodermic injection device to be compact, low cost and have the required shelf life for implementation in disposable single use applications, for example.

The propulsion unit according to this invention may be produced as a unit separate from other parts of the injection device, in particular a cartridge or ampoule containing the liquid to be injected, such that these components may be manufactured at different sites and subsequently assembled together. This enables the ampoules to be manufactured with the required accuracy and sterility by a pharmaceutical company, for example. This also enables flexibility in the packaging and dosage of the liquid to be injected which can be determined by the volume of the ampoule.

In a preferred embodiment, the ampoule may be assembled within a container holding the compressed substance under pressure. It is however also possible to provide the ampoule in a container portion that is subsequently assembled to a container portion in which the compressible substance is contained.

The propulsion system may also be integral part of the injection device in which the liquid to be injected is also contained.

In certain embodiments, the compressed substance may be separated from the liquid to be injected by a breakable wall or a partition that is broken on actuation of the device to enable transmission of pressure from the compressed substance to the liquid to be injected.

In other embodiments, the compressed substance may be separated from the liquid to be injected by a piston or other movable member that is retained to the container portion holding the substance under pressure and may be released, for example by breaking retaining means, to liberate the piston and propel the liquid to be injected. The retaining means may, for example, be in the form of a rod attached to the piston and extending to a rear end of the container portion by the compressible substance.

In yet another embodiment, the liquid to be injected and compressed substance may be separated by a movable wall or free-floating piston, the pressure in the container being maintained by plugging an orifice or passage either between the compressible substance and the liquid to be injected, or in the nozzle through which the liquid to be injected exits.

In view of the high pressures that may be attained by the present invention, and therefore the high speed of the liquid jet produced, the jet may pierce the skin of a patient without the need for a needle in an effective, reliable and painless manner.

Depending on the application and depth of injection, it is also possible to provide with the present invention a skin piercing member that pierces the patient's skin on actuation of the device as the liberation of the pressure of the compressed substance presses on the skin piercing member. Elastic buffer means retract the piercing end of the skin piercing member into the applicator end of the device once the pressure drops during injection, such that the risk of contamination by the needle is avoided. In such embodiments, the pressure generated by the energy source could be lower than a needless device in view of the piercing of the skin by the needle prior to injection.

The skin piercing member could also form the outlet nozzle for the liquid to be injected.

A separating or pressure transmitting member, such as a piston, a membrane, a deformable wall or a breakable partition may be arranged between a portion of the container comprising the liquid to be injected and a portion comprising the compressible substance.

If the pressure transmitting member is a piston, the retaining means may be in the form of a piston retaining rod, said rod extending from the piston to an attachment portion of the device. An anchoring portion of the rod may be fixed to the attachment portion of the container by crimping the attachment portion on the rod, by welding, by coining or by other mechanical means. Crimping of the attachment portion on the anchor portion of the rod is advantageous because of its simplicity and the excellent sealing it provides of the rear end of the device. The rod may comprise a rupture zone enabling separation of the anchoring portion from the rest of the rod to free the piston.

The rupture zone may be weakened and/or rendered less ductile, such that the rod breaks in this zone on being bent. It is also possible, for example, to weaken the rupture zone by provision of a groove, holes or an indent. The rupture zone may be rendered less ductile by a tempering process, particularly if the rod is made of steel alloy. The tempering may be effected by local heating, by laser, ultra-sound, electromagnetic induction or other means, followed by cooling.

The retaining means may be in the form of a plug blocking the orifice of the nozzle portion. The plug may be of a material that may be decomposed by external means such as heat or ultrasound, for example a wax or paraffin plug that may be removed by locally heating the injection device. The plug may also be a mechanical member such as steel wire retractable from the orifice. In an embodiment, the floating piston or deformable wall moves once the orifice is unblocked due to the drop in pressure in the container portion comprising the liquid to be injected.

In another embodiment, the portions comprising the liquid to be injected and the compressible substance are separated by a passage of reduced section which may be blocked by different means, either by mechanical means or by means that may be disintegrated, for example by heat, as in the case of paraffin or wax, such means forming the aforementioned retaining means.

In these embodiments, the liquid to be injected is at atmospheric pressure until the passage between the container portions is freed from the retaining means and the pressure transmitting member between these portions is propelled by expansion of the compressible substance.

In another embodiment, the portion containing the liquid to be injected is surrounded by a deformable wall arranged inside the container portion containing the compressible substance, and the retaining means comprise a plug closing the orifice of the nozzle portion. Once the retaining means are removed, the deformable wall of the container portion containing the liquid to be injected is crushed under the pressure of the compressible substance.

In another embodiment, the portion containing the compressible substance is arranged inside the portion containing the liquid to be injected and once the retaining means are removed, the deformable wall of the container portion containing the compressible substance expands within the portion containing the liquid to be injected so as to propel the liquid out of the device through the orifice of the nozzle. The compressible substance thus expands to occupy the volume of the container portion containing the liquid to be injected. The compressible substance may occupy a continuous volume or a plurality of separate volumes, for example in form of a plurality of capsules or a large plurality of micro-capsules. These capsules may, for example, comprise a membrane surrounding the compressible substance, such as a visco-elastic liquid like polysiloxane, or simply consist of a solid substance, such as rubber.

The container may be made of metal, for example made of stainless steel, which may be provided with a precious metal layer on its inside surface (for example gold, platinum, palladium) or with a polymer such as Teflon. The inside layer assists in maintaining the purity and sterility of the medicament. In addition, the inside layer facilitates sliding of the piston, if applicable, and improves sealing. Sealing between container portions containing the compressible substance and the liquid to be injected may also be improved by providing the inside of the container portion containing the compressible substance with a polymeric or elastic layer, for example rubber, surrounding the compressible substance. It should be noted that polysiloxane oils are very advantageous with respect to a gas, on the one hand, due to their viscosity which may be very high depending on the molecular weight of the oil, thereby reducing the demands on sealing, and on the other hand, a large portion of the stored compression energy may be transformed into work.

The nozzle portion may comprise a separate member mounted in or to the container, or may be integrally formed with the wall of the container or at least the container portion containing the liquid to be injected.

The orifice of the nozzle portion may have a diameter in the order of 10 to 80 microns, at least over a defined length, such that the liquid jet remains coherent for a few millimetres after exiting the nozzle. If the displacement of the piston between the beginning and end of the injection corresponds to a variation in volume of the compressible substance of 7.5 %, this corresponds to a pressure variation of 1000 bars for monomer hexamethylsiloxane. A pressure of this order combined with a very fine nozzle orifice enables the production of a supersonic jet for liquid injections through skin in an extremely reliable and painless manner. Moreover, the supersonic shock wave causes degradation of the jet in droplets a few millimetres from the nozzle, thereby increasing the safety of the device. The jet could of course also be produced at subsonic speeds depending on the injection needs and requirements.

The container portion containing the liquid to be injected may have a smaller diameter than the container portion containing the compressible substance, the piston comprising a first portion and a second portion having diameters adapted to diameters of the respective container portions, such that there is a pressure multiplication substantially equal to the ratio of the cross-sectional areas of these container portions.

The compressible substance may be compressed by filling the container under pressure, or by filling it at atmospheric pressure or at low pressure and subsequently deforming the container portion containing the compressible substance, thereby reducing its volume.

In embodiments where first and second compressible substances are present, these may be provided in different sections of the container, separated by a movable or breakable portion, or by a reduced section passage blocked by a plug prior to use. During use, the first compressible substance produces a high pressure jet to pierce a patient's skin in an initial injection phase. Subsequently, the lower pressure second compressible substance completes injection.

Further objects and advantageous aspects of the invention will be apparent from the following description, claims and accompanying drawings.

### Brief Description of the Drawings

Fig. 1 is a longitudinal section of an injection device prior to use according to a first embodiment of the invention;
Fig. 2 is a longitudinal section of the first embodiment during use;
Fig. 3 is a view showing the use by a patient of an injection device according to the invention;
Fig. 4 is a longitudinal section of a variant of the first embodiment;
Fig. 5 is a partial longitudinal section of part of a retaining rod for the variant of Fig. 4, showing the rupture zone thereof;
Fig. 6 is a section through line VI-VI of Fig. 5;
Fig. 7 is a longitudinal section of a part of another variant of a retaining rod, showing the rupture zone thereof;
Fig. 8 is a section through line VIII-VIII of Fig. 7;
Fig. 9 is a longitudinal section of a second embodiment of a device according to the invention;
Fig. 10 is a view of a variant of the first embodiment;
Fig. 11 is a section through line XI-XI of Fig. 10;
Fig. 12 is a view of another variant of the first embodiment;
Fig. 13 is a partial longitudinal section of the embodiment of Fig. 12 with an actuator button and a support for positioning the front end of the device against the skin of a patient;
Fig. 14 is a partial longitudinal section showing a retaining means of a piston;
Fig. 15 is a longitudinal section of a part of the device showing another variant of a retaining means;
Fig. 16 is a longitudinal section similar to that Fig. 15 after actuation of the piston;
Fig. 17 is a longitudinal section of a third embodiment of an injection device according to the invention;
Fig. 18 is a longitudinal section of a fourth embodiment of a injection device according to the invention;
Fig. 18a is a longitudinal section of a variant of the fourth embodiment of an injection device;
Fig. 18b is a detailed section of the nozzle portion of the device shown in Fig. 18a;
Fig. 19 is a longitudinal section of a fifth embodiment of an injection device according to the invention;
Fig. 20 is a longitudinal section of a variant of the fifth embodiment of an injection device according to the invention;
Fig. 21 is a longitudinal section of a sixth embodiment of an injection device according to the invention;
Fig. 22 is a longitudinal section of a seventh embodiment of an injection device according to the invention;
Fig. 23 is a longitudinal section of an eighth embodiment of an injection device according to the invention;
Fig. 24 is a longitudinal section of a ninth embodiment of an injection device according to the invention;
Fig. 25 is a longitudinal section of a tenth embodiment of an injection device according to the invention, prior to use;
Fig. 26 is a longitudinal section of the tenth embodiment, during use;
Fig. 27 is a longitudinal section of a variant of the tenth embodiment of an injection device according to the invention, prior to use;
Fig. 28 is a longitudinal section of the embodiment of Fig. 27, during use;
Fig. 29 is a longitudinal section of an eleventh embodiment of an injection device according to the invention;
Fig. 30 is a longitudinal section of a twelfth embodiment of an injection device according to the invention;
Fig. 31 is a longitudinal section of a variant of the twelfth embodiment of an injection device according to the invention;
Fig. 32 is a longitudinal section of a thirteenth embodiment of an injection device according to the invention, prior to use;
Fig. 33 is a longitudinal section of the thirteenth embodiment of an injection device according to the invention, during use;
Fig. 34 is a longitudinal section of a fourteenth embodiment of an injection device according to the invention;
Fig. 35 is a longitudinal section of a variant of the third embodiment of an injection device according to the invention;
Figures 36 and 37 are longitudinal sections of a fifteenth embodiment of an injection device according to the invention, prior to use and during use, respectively;
Fig. 38 is a longitudinal section of a variant of the fifteenth embodiment of an injection device according to the invention;
Fig. 39 is a longitudinal section of a sixteenth embodiment of an injection device according to the invention, during an initial injection phase;
Fig. 40 is a longitudinal section of the sixteenth embodiment during a final phase of injection;
Fig. 41 is a graph showing the injection pressure versus time of embodiments of an injection device according to Figures 39 to 43 ;
Fig. 42 is a longitudinal section of a seventeenth embodiment of an injection device according to the invention in an initial injection phase;
Fig. 43 is a longitudinal section of the seventeenth embodiment during the final injection phase;
Fig. 44 is a longitudinal section of an eighteenth embodiment of an injection device according to the invention, prior to use;
Fig. 45 is a longitudinal section of the eighteenth embodiment, during use;
Fig. 46 is a longitudinal section of a variant of the eighteenth embodiment, after use;
Fig. 47 is a detailed section view of an applicator end of an injection device according to the invention, during hypodermic injection of a patient;
Fig. 48 is a longitudinal section of part of an embodiment of an injection device according to the invention with dosage adjustment means;
Fig. 49 is a partial longitudinal section of a nineteenth embodiment of an injection device according to this invention prior to use;
Fig. 50 is a partial longitudinal section of a another variant of a nozzle portion;
Fig. 51 is a longitudinal section of a rechargeable propulsion unit of a twentieth embodiment of an injection device according to this invention, the injection device having a multi-use propulsion unit for use with single-use medicament capsules;
Fig. 52 is a longitudinal section of a capsule of the twentieth embodiment for assembly to the propulsion unit;
Fig. 53 is a longitudinal section of a twenty-first embodiment of an injection device according to this invention, with a single-use capsule containing the compressible substance and the liquid to be injected mountable in a pressure generating unit;
Fig. 54 is a longitudinal section of the single-use capsule of the embodiment of figure 53;
Fig. 55 is a longitudinal section of the pressure generating unit of the embodiment of figure 53;
Fig. 56 is a longitudinal section of a variant of the embodiment of figure 53, in which the compressible substance is mounted in the pressure generating unit and the single-use capsule contains the liquid to be injected.

### Detailed Description of the Invention

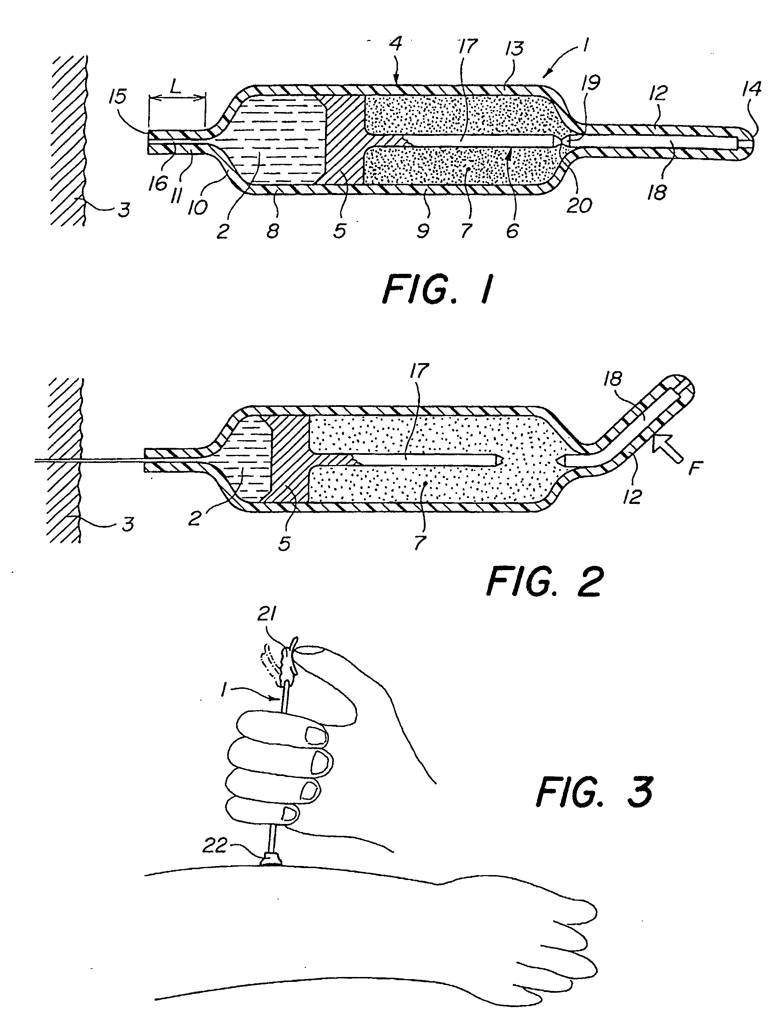

Referring to Figures 1 to 3, an injection device 1 for the administration of a liquid 2 under the skin 3 of a human or animal patient, comprises a container 4, a pressure transmitting member in the form of a piston 5, a pressure retaining means 6 and a compressible substance 7. The container 4 comprises a portion 8 containing the liquid to be injected and a portion 9 containing the compressible substance. The container portion 9, the compressible substance 7, the piston 5 and the pressure retaining means 6 form part of a propulsion system of the device for propelling the liquid to be injected, whereby the compressible substance 7 under pressure is a source of potential energy..

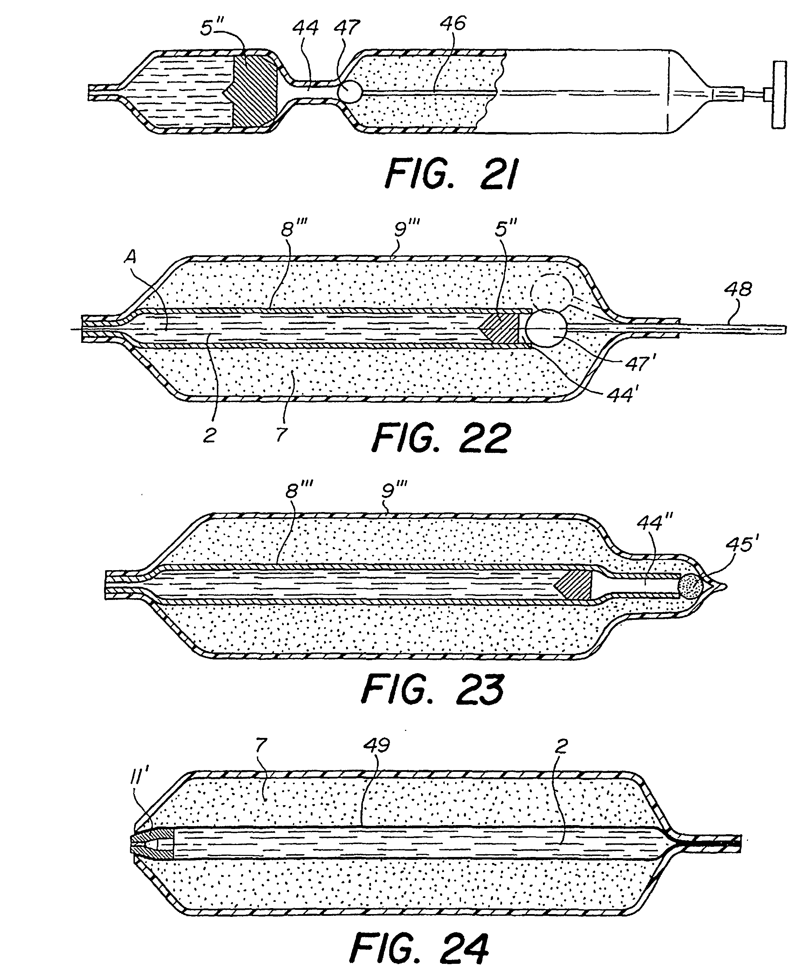

The device further comprises a collar portion 10 and a nozzle portion 11 which may be integrally formed with the container portion 8 containing the liquid to be injected. The nozzle portion may also comprise or be part of a separate piece mounted in or to the container as shown in Fig. 24 under reference 11'. The device may also comprise an attachment portion 12 integrally formed with the outer wall 13 of the container. The wall 13 of the container thus extends, in this particular embodiment, integrally from a rear end 14 to a front or application end 15.

The nozzle portion 11 has an orifice 16 which may have a diameter in the order of 5 to 100 microns, but which is preferably in the range of 20 to 50 microns. The orifice extends over a length L which is preferably between about two to five times the diameter of the orifice. The ratio between the length L and the orifice diameter enables the production of a liquid jet that remains coherent over a distance sufficient to ensure reliable hypodermic injection, but which destabilizes after a few millimetres, thereby making the jet harmless. In other words, the ratio between the length and diameter of the orifice enables the coherence of the jet to be regulated, such that it is sufficiently coherent for effective and reliable hypodermic injection without being too coherent for safety reasons.

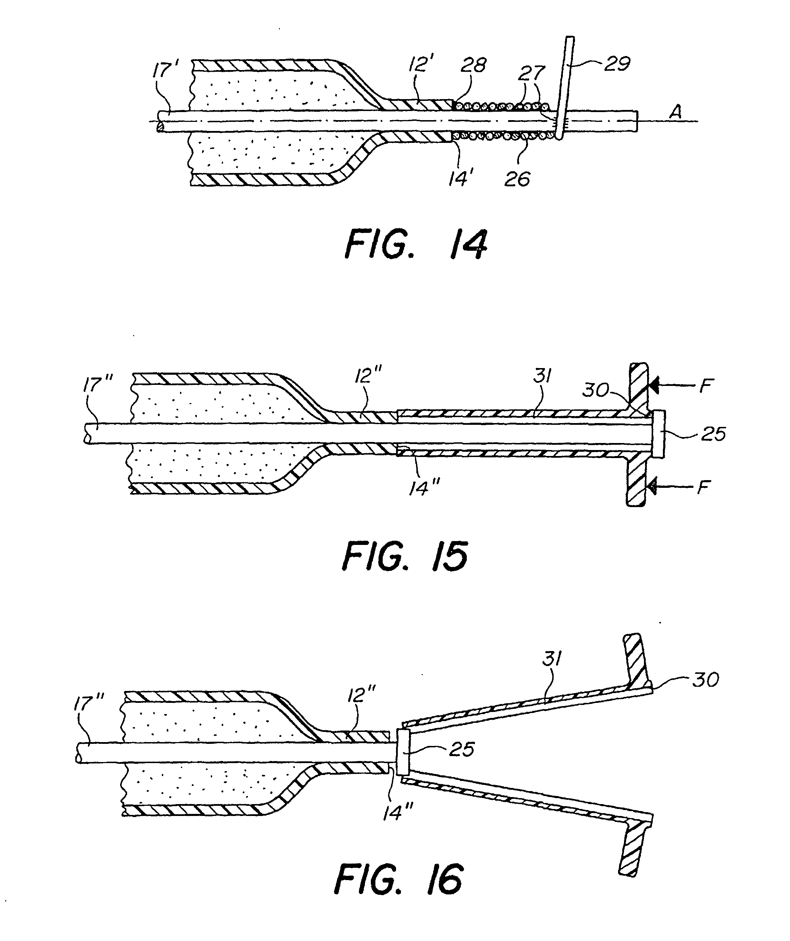

The retaining means 6 comprise, in this embodiment, a retaining rod 17 attached or integrally formed with the piston 5 and extending to an anchoring portion 18 fixed to the attachment portion 12 of the container 4. The anchoring portion of the rod may be fixed to the attachment portion by crimping or other means such as coining, welding or by the provision of a ledge 25, as shown in Fig. 15.

Between the piston 5 and the anchoring portion 18, the rod is provided with a rupture zone 19 to enable liberation of the piston 5 by rupture of the rod in this zone. The rupture zone comprises a groove 20 to reduce the cross section of the rod. The rupture zone may also be made more fragile by localised tempering. The tempering may be effected by local heating, for example by laser, ultra-sound or electromagnetic induction, followed by rapid cooling. To this effect, the retaining rod 17 is preferably made of steel. It is also possible to make the piston and rod in glass or other materials, such as carbon-reinforced epoxy with sufficient fragility to be broken when the rod is mechanically actuated (twisting or bending) by a user. In this embodiment, the rupture zone 20 is proximate the attachment portion of the container, as shown in Figures 2 and 3, such that plastic bending of the attachment portion causes the rod to break in the rupture zone.

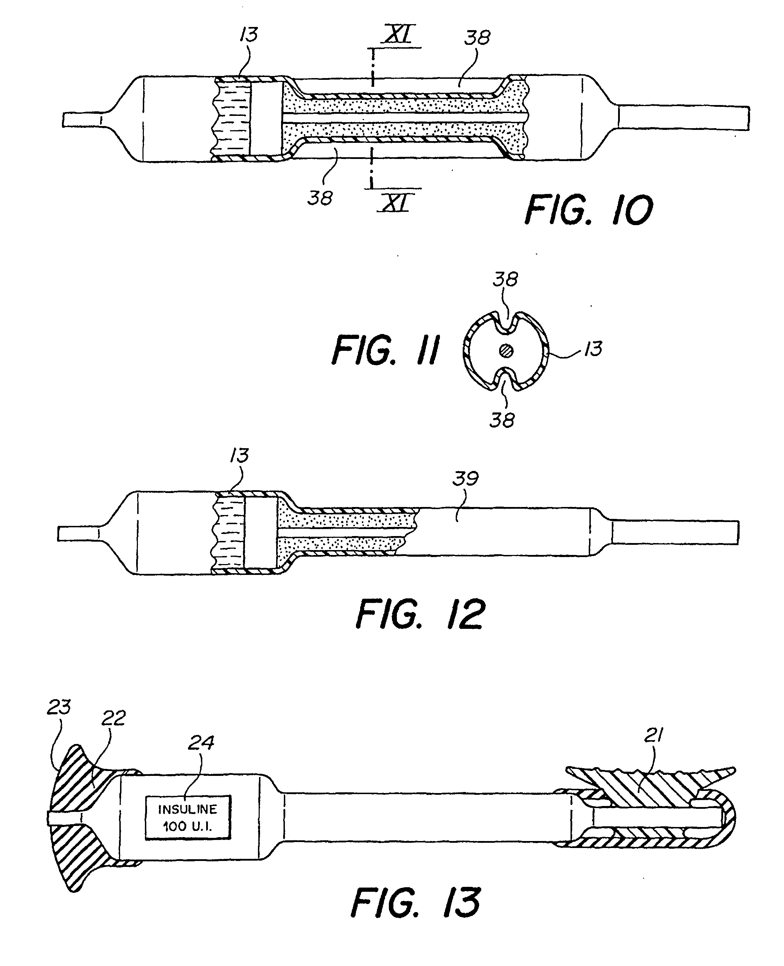

To facilitate this bending, the injection device may be provided with a pushing member 21, as shown in Figures 3 and 13, inserted over the attachment portion 12 at the rear end of the injection device.

The plastic permanent deformation of the attachment portion 12 has the advantage of providing a clear indication to the user that the disposable injection device has been used. The injection device comprises a support 22, as shown in Figures 3 and 13, for example made of plastic, mounted on the front or application end of the device and having a pressure application surface 23, to improve the positioning of the extremity 15 of the nozzle portion 11 as well as increasing comfort to the user.

To identify the product to be injected, the device may further comprise an identification patch 24, as shown in Fig. 13, indicating the type of product, its composition, quantity, etc.

The substance may advantageously comprise soft matter, such as a polysiloxane oil. Soft matter has the ability to store a large amount of potential energy through elastic molecular compression, for example up to 100 times more energy than a conventional metal spring occupying the same volume. The molecules of soft matter behave as three-dimensional springs, and the stored energy is equal to the sum of the molecular cohesion energy of about 4 · 10⁻²¹ joules per molecule which corresponds to the thermal energy K_{B}T at 20° C, where K_{B} is Boltzmans constant, and T is temperature in Kelvin. The elastic property of soft matter is particularly advantageous to the present invention since it allows the injection device to be compact, cost-effective, and comprise few components. Depending on the molecular weight, polysiloxanes typically have volumetric compressibility values (dVN at a given pressure) three to four times greater than the volumetric compressibility of water. While polysiloxanes are a preferred soft matter for use in the present invention, other soft matter substances may also be used. The properties of soft matter are known and described, for example, in the reference "Review of Modern Physics", Nobel Lecture in Physics, vol. 64, p. 645.

Polysiloxane oils are limpid, clear, odourless, insipid, visco-elastic liquids resistant to high and low temperature and which are low-cost. They are neither toxic nor dangerous from the physiological point of view and may be used in dermatological and cosmetic applications. Polysiloxane oils have a low viscosity variation as a function of pressure which advantageously facilitates fluid exchange, but they have a high surface tension such that they are non-miscible with water solutions. Polysiloxane oils also have lubricating properties between metals and polymers and rubber, which advantageously facilitates sliding between mobile members.

The family of polysiloxane oils comprises, inter alia, the following substances:
- polymethylhydrogensiloxane
- polydimethylsiloxane
- polytrimethylsiloxane
- hexamethylcyclotrisiloxane
- decamethyltetrasiloxane
- hexamethyldisiloxane (H 7310 - Witheco)
- octamethyltrisiloxan (O 9816 - Witheco).

An advantageous property of polysiloxane oils is the reduction of viscosity with shear velocity which enables rapid flow of such oils through small orifices. Polysiloxane oils may have viscosities ranging from 0.6 to 10⁷ centistokes depending on molecular weight. This property enables the oil to be chosen according to the requirements of the embodiment, in particular embodiments that require flow of the compressible substance through passages of small cross sections, as is the case for the embodiments shown in Figures 19 to 23 and 34, which may comprise a polysiloxane oil of low viscosity. The other embodiments, particularly those comprising a piston, may be provided with polysiloxane oils of high viscosity, which have the consistency of a gel, thus reducing the sealing requirements or enabling higher pressures.

The compressible substance may also comprise an elastic solid, such as vulcanised silicon rubber, for example of the type SilGel® 6/2 manufactured by Wacker-Chemie, having good compressibility properties.

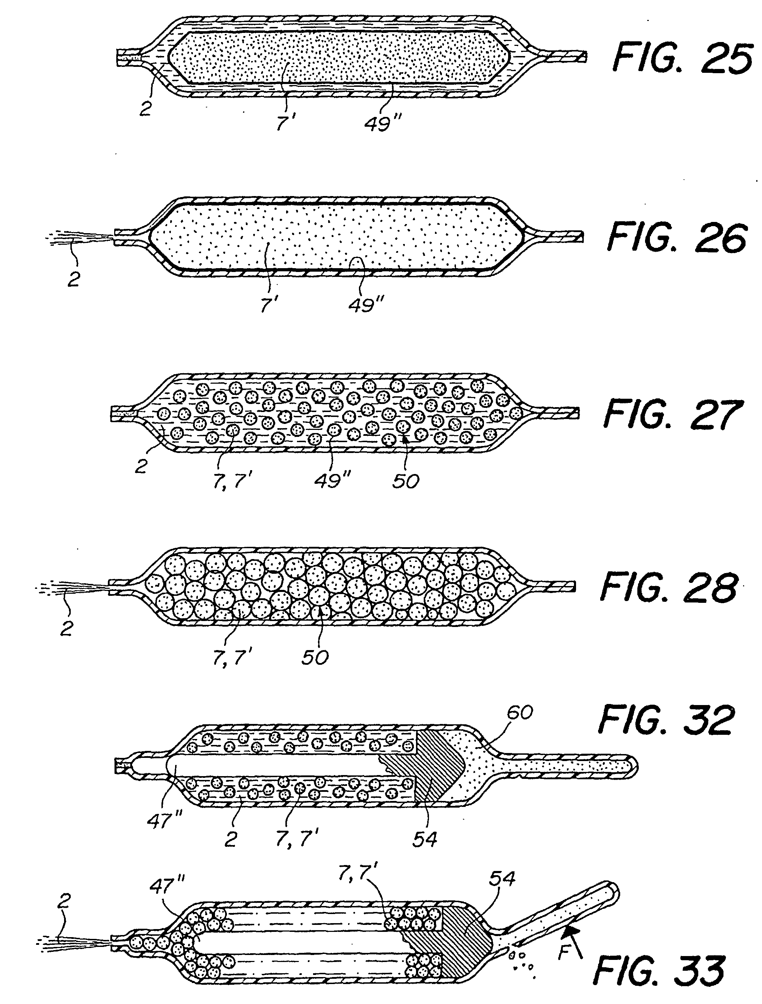

Use of a solid compressible substance is advantageous in certain embodiments, such as those of Figures 25 to 28 which will be described in greater detail further on.

As an example, monomer hexamethylsiloxane (CH₃)₆ SiO may be elastically compressed under a pressure of approximately 2000 bars with a volume reduction of about 15%. If the volume of the liquid to be injected is 0.1 ml, and the minimum pressure at the end of injection is chosen to be 1000 bars, the non-compressed volume of polysiloxane is 1.3 ml. The device according to the invention is not only extremely compact, but enables the injection of liquid at pressures well above those available in conventional systems, which makes possible the production of a very fine jet that can surpass supersonic speed. Very reliable and safe hypodermic injection can thus be effected with the present invention.

For example, at 1000 bars pressure, the liquid to be injected can be propelled through nozzle orifices having diameters around 30-60 µm with sufficient speed to pierce a patients skin, and whereby injection time is slow enough to enable the injected liquid to diffuse in the surrounding tissue thus reducing injection pain. In conventional devices, the nozzle orifice must have a much larger diameter in view of the lower injection pressure, with the consequence that injection time is reduced and the injected liquid collects locally in the patient's tissue thus causing pain.

Moreover, the injection device according to the invention comprises very few parts which leads to low-cost production which is well adapted to disposable products that guarantee sterility, ease of storage and distribution, in addition to simple and reliable use.

Fig. 14 shows another variant of retaining means in which the retaining rod 17' is fixed to the attachment portion 12' by a helicoidal wire 26 that is welded to the rod at welding points 27, 28. The rod 17' does not comprise a rupture zone and is slidably mounted in the attachment portion 12'. Actuation of the device is effected by applying torque on the spring actuation extremity 29 around the longitudinal axis A to break the micro-welds 27, 28, thus liberating the rod 17'.

Fig. 15 shows another variant of retaining means in which the retaining rod 17" is slidably mounted in the attachment portion 12" and retained by engagement of a ledge 25 against an extremity 30 of a split tube 31 which abuts at its other extremity against the end 14" of the injection device. An axial force F applied on the lateral extensions 32 causes rotation of the tube portions 31, thereby disengaging the ledge 25 from the extremity 30, as shown in Fig. 16.

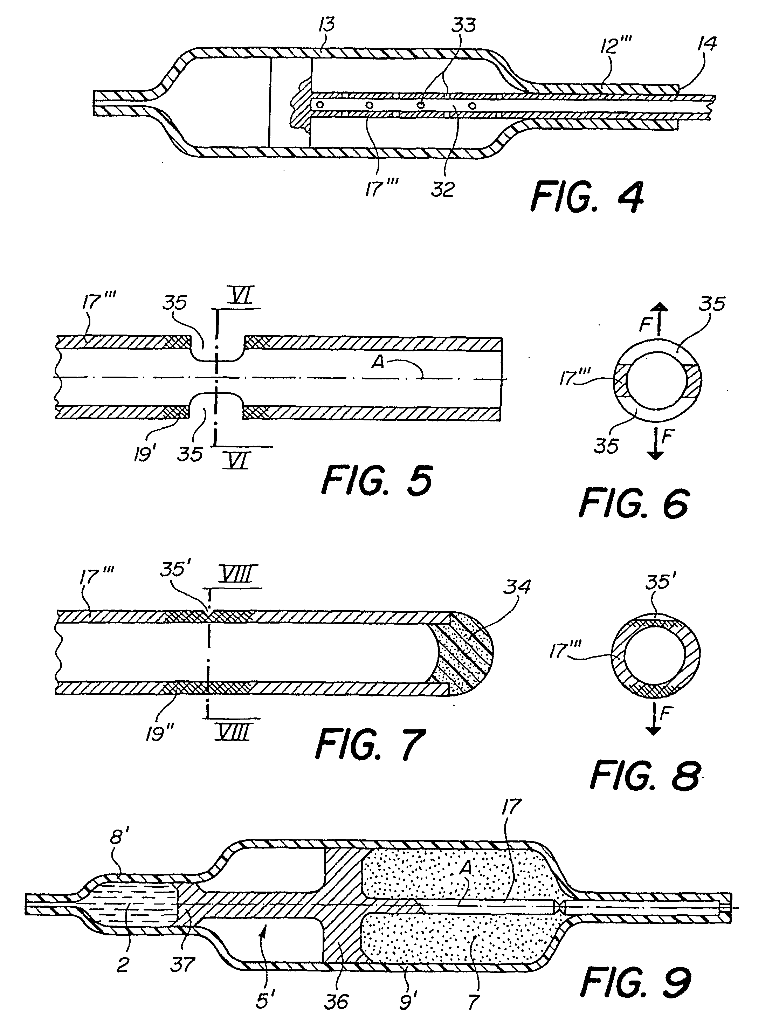

Fig. 4 shows another embodiment in which the retaining rod 17"' comprises a central passage 32 and lateral holes 33 to enable filling of the container portion containing the compressible substance from the rear end 14 of the device. After the filling operation, the rear end of the passage 32 may be closed by a solder drop or glue 34, as illustrated in Fig. 7 or the tube 17"' may be crushed by a crimping or crushing operation on the attachment portion 12"'.

A rod in the form of a tube 17"' may have a rupture zone 19', 19" weakened by the provision of lateral holes 35, as shown in Figures 5 and 6, in a tempered zone which is thus fragile, or by other weakening means, such as a groove 35', as shown in Figures 7 and 8. In the variant shown in Figures 5 and 6, the rod is broken by applying a force transverse to the longitudinal axis A on one or the other sides thereof provided with a hole, whereas in the variant of Figures 7 and 8, the rod is broken by applying a force on the side thereof provided with the groove 35'.

In Fig. 9, a third embodiment of the invention comprises a pressure multiplying system. The pressure multiplying system is achieved by providing a first portion 36 of the piston 5' with a greater surface area (in cross section), in contact with the compressible substance 7, to the surface area (in cross section) of a second portion 37 of the piston in contact with the liquid to be injected 2. The container portion containing the compressible substance 9' thus has a larger diameter than the container portion containing the liquid to be injected 8'. The pressure multiplication is equal to the ratio between the surfaces of the piston portions 36, 37 taken in orthogonal cross-section with respect to the longitudinal axis A. The pressure multiplication enables the device to be shortened, the injection pressure to be increased, or the compression of the compressible substance decreased, thus providing a larger field of use of the device.

In Figures 10 to 12, the container portion containing the compressible substance comprises indents 38 or a reduced diameter 39 effected after filling this portion with the compressible substance. A volume reduction of this portion by permanent deformation of the wall 13 may take many different shapes, the important aspect being to reduce the volume so as to pressurise the compressible substance. The aforementioned method of pressurising the compressible substance may also be used in the other embodiments discussed herein. The latter allows the container portion containing the compressible substance to be filled at low pressure, thus facilitating the filling operations and other operations for producing the device. As already mentioned, if the compressible substance is a polysiloxane, the volume may be reduced by approximately 15% to generate 2000 bars of pressure.

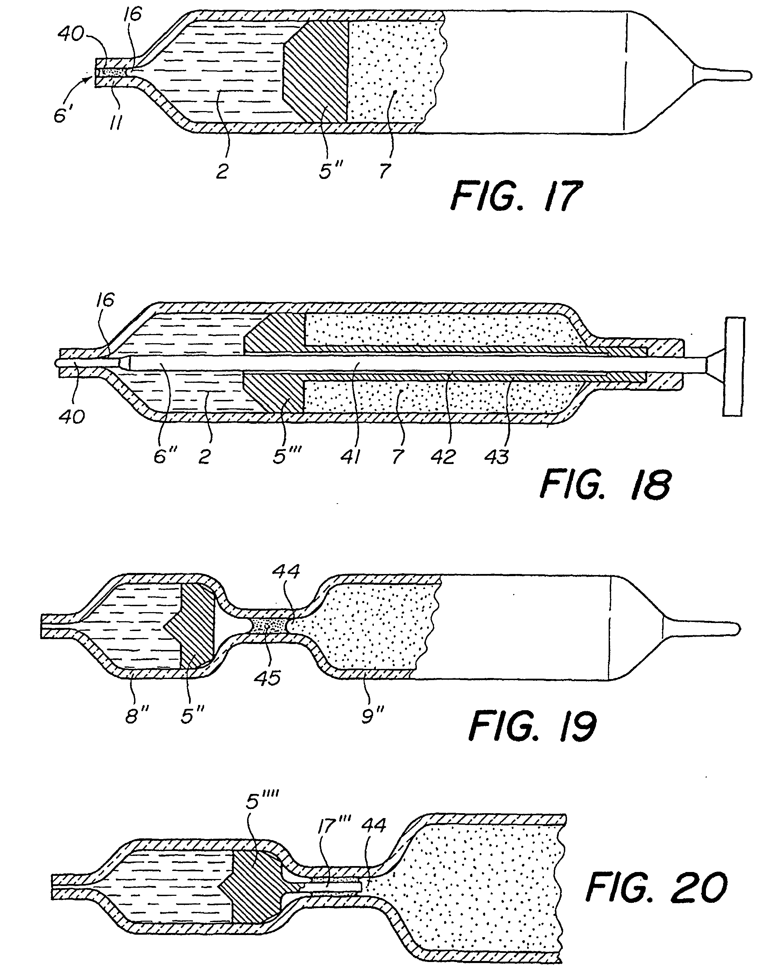

In Fig. 17, another embodiment is shown in which the retaining means 6' comprises a plug 40 closing the orifice 16 of the nozzle portion 11, such that the pressure of the liquid to be injected 2 is equal to the pressure of the compressible substance 7 and that the piston 5" separating the liquid and the substance is floatably mounted therebetween.

The plug may for example be made of a material that decomposes under the effect of external solicitation, for example a meltable material may be removed by local heating of the nozzle portion 11 during use. Bees' wax or paraffin are examples of meltable materials which may be used in the present invention. As the orifice 16 has a very small diameter in the order of 50 µm or less, the wax plug suffices to block the orifice and resist to pressures up to 4000 bars.

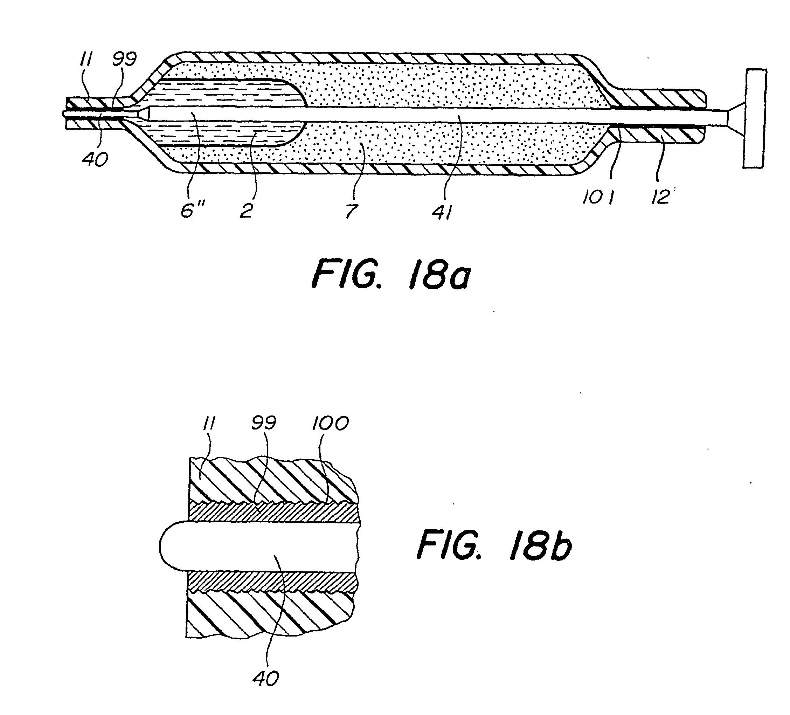

Fig. 18 shows another embodiment in which the piston 5''' is mounted more or less floatably, such that the liquid to be injected 2 is at the same pressure as the compressible substance 7. The retaining means 6" comprise a plug 40 on a rod 41 extending to the rear end of the injection device. When the rod is pulled backwards, the plug 40 liberates the orifice 16 and the piston 5"' is propelled by the compressible substance 7. The piston 5''' is provided with a passage 42 for the rod 41. The passage 42 may be sealed, yet allow the piston to slide along the rod. The piston 5"' may also be attached to a tube 43 which extends up to the rear end of the device to improve sealing.

Fig. 18a shows an embodiment similar to that of Fig. 18, except that there is no piston separating the compressible substance 7 from the liquid to be injected 2. The compressible substance is a solid, such as vulcanized rubber or very high molecular weight polysiloxane that does not mix or create a solution with the liquid to be injected.

To obtain an effective sealing between the plug 40 and a wall of the container nozzle portion, an insert 99 is provided, or a layer is plated or otherwise deposited on the inside of the nozzle portion 11. The insert or layer 99 is made of a ductile material such as gold or an alloy thereof. The inner layer 99 of the nozzle portion may also be provided in the form of a tubular insert, the tip of the nozzle portion 11 and insert 99 subsequently crimped on the plug 40. The plastic deformation of the ductile metal around the plug during the crimping operation ensures a particularly effective seal that is able to withstand very high pressures in the range of 1000 bars or more inside the container. The relatively small diameter of the plug 40 and the relatively low friction coefficient of ductile insert or layer enables the plug to be retracted with a reasonable pulling force on the handle of the rod 41. The plug 40 is represented as a pin extending from a larger diameter rod 41, but in view of the relatively small orifice, is preferably a fine wire, for example having a diameter of 50 microns, of high tensile steel or composite material extending to the handle 98 and having a substantially constant diameter. It is also possible to provide the plug 40 and ductile insert 99 positioned just behind the orifice in a portion having a larger diameter than the outlet orifice. In order to obtain a good adhesion and sealing between the insert 99 and the inner surface 100 of the nozzle portion, the inner surface of the nozzle portion is preferably provided with a certain roughness allowing the insert material to plasticly flow into the interstices during crimping of the nozzle portion tip around the plug 40. This improves adhesion of the insert to the nozzle and ensures that the insert remains in place even under the high pressure during operation of the device.

The rear end 12 also comprises an insert 101 of ductile material such as gold or an alloy thereof for the same reason as the nozzle portion insert. The crimping may be made with less crushing force than the nozzle portion in order to reduce the frictional force needed to slide the rod or wire 42 on actuation of the device. This is because the compressible substance may have a higher viscosity than the liquid to be injected, thus reducing the sealing requirements.

Fig. 19 shows another embodiment in which the piston 5" is floatably mounted as in the embodiment of Fig. 17, but the container portion 8" containing the liquid to be injected and the container portion 9" containing the compressible substance communicate through a reduced section passage 44 which is blocked by retaining means 45, prior to use. The retaining means may be a plug made of meltable material, such as paraffin, and which is removed by local heating, as shown in Fig. 19. The retaining means may also comprise a rod 17" which is inserted in the passage 44 and which extends from the piston 5"", as shown in Fig. 20. The rod 17"" is retained by a meltable substance, for example paraffin, which may be melted by local heating. It is also conceivable to fix the rod by crimping or by other mechanical means, and to break the rod during actuation of the device by bending the container portion containing the liquid compressible substance with respect to the container portion containing the liquid to be injected.

The plug may also comprise a rod 46 provided with a plug portion 47 at its extremity blocking the passage 44, as shown in Fig. 21. The rod is pulled back to disengage the passage 44, thereby actuating the injection.

Instead of having juxtaposed container portions, it is also possible to position the container portion containing the liquid to be injected inside or outside the container portion containing the compressible substance. Since in most applications, the compressible substance occupies a greater volume than the liquid to be injected, the container portion containing the liquid to be injected is preferably arranged inside the container portion containing the compressible substance, as shown in Figures 22 and 23, the container portion containing the liquid to be injected being designated by reference number 8"' and the container portion containing the compressible substance being designated by the reference number 9"'. In these two embodiments, a piston is slidably mounted in the container portion containing the liquid to be injected, which communicates with the container portion containing the compressible substance by a passage 44" which is blocked by retaining means that may be formed from meltable material, such as paraffin, as shown in Fig. 23, or which may be formed by a mechanical plug that may be disengaged, for example by rotation of the rod 48 around an axis perpendicular to a plane comprising the longitudinal axis A or, as in the variant shown in Fig. 21, by retracting the rod 46 in the longitudinal direction.

In Fig. 24, the pressure transmitting member comprises a deformable wall 49 which separates the liquid to be injected from the compressible substance 7, both being substantially at the same pressure. In this embodiment, the retaining means comprises a plug in the orifice of the nozzle portion which may for example be similar to the plug described in relation to the embodiment of Fig. 17. When disengaging the orifice during actuation of the device, the deformable wall 49 collapses under the pressure of the compressible substance 7. The wall 49 may be a thin metallic tube or made of a plastic or rubber material.

In Fig. 25, the container portion containing the compressible substance 7' is inside the container portion containing the liquid to be injected 2, such that when the orifice of the nozzle portion is opened, the compressible substance expands elastically to fill the volume of the container portion containing the liquid to be injected, thereby expulsing the liquid through the orifice of the nozzle portion, as shown in Fig. 26.

The compressible substance 7' may be a polysiloxane closed within a membrane 49" that is elastically or plastically deformable, or it may be a solid material such as vulcanised silicon rubber. In such an embodiment, the pressure transmitting member may be considered as the exterior surface or wall of the rubber member.

Figures 27 and 28 show an embodiment similar to those of Figures 25 and 26, respectively, except that the compressible substance does not occupy a continuous volume, but is divided in a plurality of capsules enclosing polysiloxane or other compressible liquids, or a plurality of rubber balls or other solid compressible substances, within the liquid to be injected prior to use, as shown in Fig. 27. On disengagement of the orifice of the nozzle, the microcapsules or balls expand and expulse the liquid to be injected, as shown in Fig. 28. In this embodiment, the pressure transmitting member may be considered as a multitude of walls or outer surfaces of the capsules or balls 50, respectively.

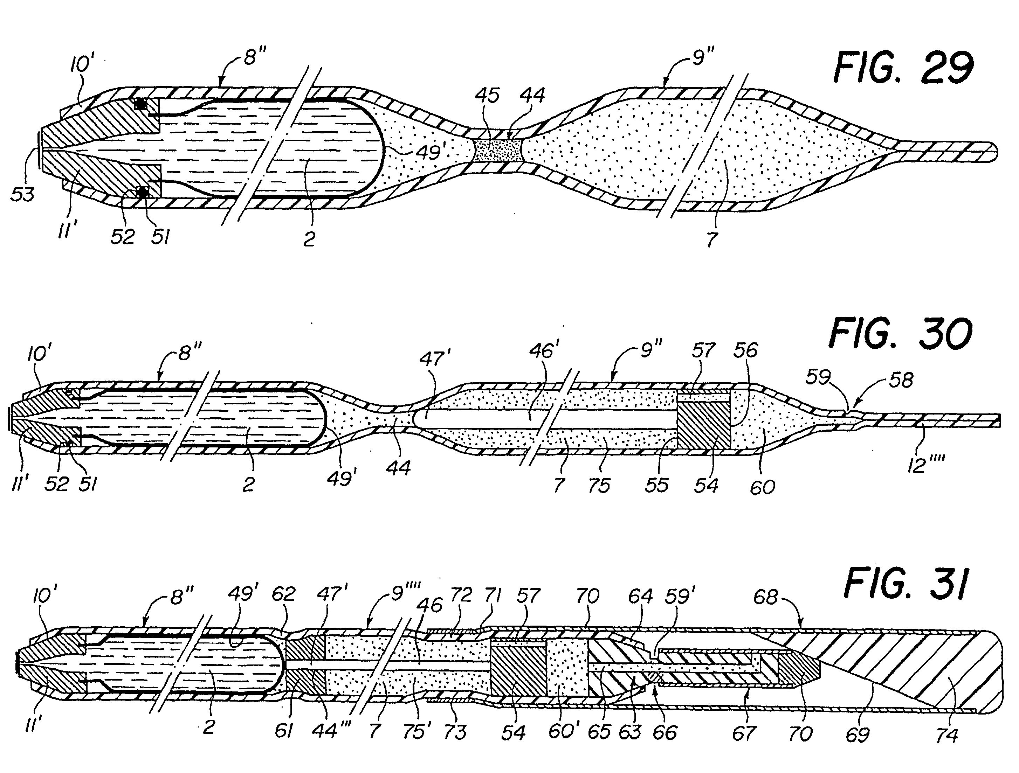

Fig. 29 shows an embodiment similar to that of Fig. 19 comprising a container portion 8" containing the liquid to be injected and a container portion 9" containing the compressible substance, communicating through a reduced section passage 44 blocked by retaining means 45. In this embodiment, the retaining means is constituted by a plug of decomposable material which may be disengaged by external solicitation such as ultrasound or local heating. This embodiment differs from that of Fig. 19, particularly in that it does not comprise a piston, the liquid to be injected 2 being surrounded by a deformable membrane 49', for example made of polyethylene. The membrane 49' may form the wall of a sterile cartridge or ampoule which also comprises the nozzle portion 11' and contains the liquid to be injected. The cartridge is assembled in the wall of the container and fixed, for example, by inward deformation (e.g. crimping) of the collar portion 10'. To ensure sealing between the nozzle portion and the interior surface of the container portion 8" containing the liquid to be injected, an O-ring seal may be provided in a groove 52 around the rear end of the nozzle portion. A protective film 53, for example of polyethylene 10 µm thick, may be glued to the front end of the nozzle portion to ensure sterility and sealing of the cartridge. Advantageously, the cartridge is filled with liquid products under conditions adapted to large volumes and guaranteeing sterility and accurate dosing for the specified use without influencing the design of other portions of the injection device. The cartridge or ampoule may subsequently be assembled in the container portion 8" containing the liquid to be injected.

Fig. 30 shows another embodiment comprising a container portion containing the liquid to be injected and a nozzle portion identical to those of Fig. 29, but with different retaining means of the compressible substance 7. The retaining means comprise a plug 47' on a rod 46' attached to a piston 54 in the container portion 9" containing the compressible substance 7. Prior to use, the plug 47' blocks the passage 44 connecting the container portions 8", 9". The compressible substance 7 which is preferably a liquid substance such as a polysiloxane described hereinabove, is maintained under pressure by the plug 47' closing the passage 44, the pressure on the front and rear sides 55, 56 of the piston 54 being equalised by one or more orifices or passages 57 traversing the piston 54. The front side of the rod 46' has a smaller surface than the rear side 56, such that the piston 54, the rod 46' and the plug 47 are subjected to a resulting force towards the front (i.e. in a direction of the passage 44), thereby ensuring that the passage 44 is blocked by the plug 47'. The volume of the compressible substance 7 in the front portion between the piston and the plug 47' has a volume sufficient to expulse the specified volume of liquid to be injected by decompression, whereas the rear portion of the container between the rear side 56 of the piston and the rear end 12"" has a very small volume, but which allows sufficient displacement of the piston 54 towards the rear end to disengage the plug 47' from the passage 44. The container is provided with a zone 58 which may be rendered fragile by tempering followed by cooling and/or by providing a groove or indent 59 in the wall of the container in this zone. The user presses on the rear portion 12"" to bend and thereby cause rupture of the container in zone 58, thereby creating a passage for the decompression of the compressible substance in the rear portion 60 of the container. The pressure drop in the rear portion 60 causes displacement of the piston towards the rear end and thereby disengagement of the passage 44. Since the orifice 57 traversing the piston 54 is very small and the viscosity of the compressible substance relatively low, the liquid to be injected 2 is propelled out of the container by expansion of the compressible substance in the front portion before the drop in pressure resulting from communication of the compressible substance with the rear portion 60 through the passage 57 is of any significance.

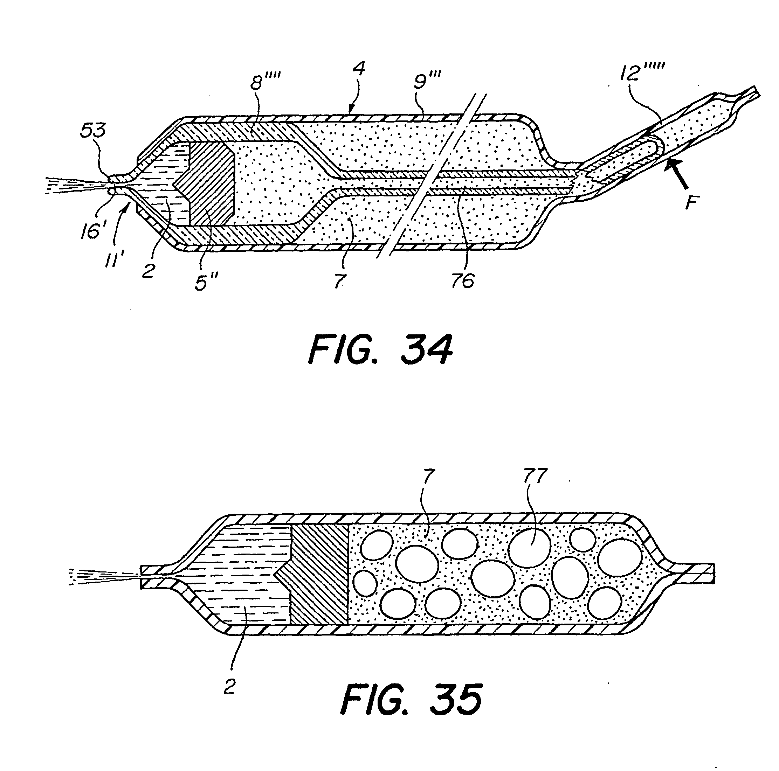

Fig. 31 shows another embodiment similar to the embodiment of Fig. 30. The container portion containing the liquid to be injected and the nozzle portion are substantially similar or identical to corresponding elements of the embodiments of Figures 29 and 30. The piston 54, rod 46' and plug 47' may also be essentially similar or identical to corresponding elements of the variant of Fig. 30. The embodiment of Fig. 31 differs from the embodiment of Fig. 30 mainly with respect to the passage portion 44' and rear portion 60' of the container. The passage 44' is provided in an insert 61 which may, for example, be made of plastic, fixed in the container, for example by a reduced section or crimping 62 of the container wall on the insert 61 at the position of the insert. The latter construction enables provision of a plug 47' extending over a certain length in the passage 44', such that actuation of the device requires displacement of the piston 54 over a few millimeters, thereby increasing the reliability and safety of the device with respect to the embodiment of Fig. 30. This embodiment thus ensures good sealing between portions containing the liquid to be injected 2 and the compressible substance 7. The rear portion 60' of the container is closed by means of a rear plug 63 which may be fixed to the inside of the portion 60' by a rear collar 64. The rear plug 63 is provided with an orifice or passage 65 interconnecting the rear portion 60' to a rupture zone 66 comprising an indent 59'. On bending a rear end portion 67 of the plug 63, the plug breaks in the rupture zone 66 such that the passage 65 communicates with the exterior of the container. The compressible substance contained in the rear portion 60' of the container is expulsed through the passage 65, such that the piston 54 is displaced towards the rear and disengages the plug 47' of the passage 44' between the container portions 8", 9"". The pressure drop in the container portion 75' containing the compressible substance 9"" due to the passage 57 in the piston 54 is minimised due to abutment of the piston 54 against the rear plug 63 during actuation, such that the flow passage towards the rear is throttled. It is to be noted that the insert 61 may be designed such that it moves as a piston towards the portion containing the liquid to be injected when the plug 47' is disengaged from the passage 44'. To this effect, liberation of the passage 44' enables radially inward crushing of the insert, such that it passes through the reduced section passage 62.

The device of Fig. 31 further comprises an actuating member 68 in the form of a pusher comprising an oblique surface 69 that may be engaged against a complementary surface 70 of the rear end portion 67 of the rear plug 63 in order to bend it and cause its rupture. The actuating member 68 comprises a tube portion 70 which may be slidably mounted on the outside of the container rear portion. A front end 71 of this member 68 is slightly inwardly inclined to engage in a slight restriction 72 of the container around which a deformable ring 73 is positioned. The restriction and the ring enable retention and positioning of the actuating member 68, prior to use. When a user pushes on the button 74, the ring 73 is displaced towards the front, while expanding elastically or plastically to move out of the restriction 72, thereby allowing sliding of the actuating member. Displacement of the ring and actuating member also provides an indication that the injection device has been used.

Figures 32 and 33 show another embodiment comprising a piston 54 with an orifice 57 extending from a container rear portion 60 to a container portion containing the liquid to be injected and the compressible substance. The piston and rear portion of this embodiment may be substantially similar or identical to the embodiment shown in Fig. 30. This embodiment differs from the embodiments of Figures 30 and 31 substantially in that the container portions containing the liquid to be injected and compressible substance are one and the same, similar to the embodiments of Figures 25 to 28. Rupture of the container rear portion as shown in Fig. 33 causes displacement of the piston 54 towards the rear end, thereby liberating the passage in the nozzle portion by disengagement of the plug 47". The compressible substance 7, 7' may be similar to those described in relation to the embodiments of Figures 27 and 28, the liquid to be injected 2 filling the remaining volume.

Fig. 34 shows another embodiment in which the container portion 8"" containing the liquid to be injected 2 is mounted inside the container portion 9"' containing the compressible substance 7 in a similar manner to the embodiments of Figures 22 and 23, except that the container portion containing the liquid to be injected is made of a material that is not very ductile, such as glass, which may be broken to allow introduction of the compressible substance in the portion containing the liquid to be injected behind the piston 5". In this embodiment, a tube 76 integrally formed with the wall of the container portion 8"" acts as a breakable partition or separating wall and as a reduced section passage when compared to the embodiments of Figures 22 and 23. The tube 76 is broken proximate its rear end by bending the rear end portion 12""' of the device. Other means for breaking the tube may however also be provided. For example, the container portion containing the liquid to be injected may be provided with an element made of magnetic material, such as a ring around the outside or a rod in the inside of the tube. The magnetic force resulting from an external magnet positioned proximate the container enables bending and consequently rupture of the tube.

In the embodiment of Fig. 34, the liquid to be injected may be filled in the portion containing the liquid to be injected in a sterile manner, but in a separate location from production of the propulsion system or assembly of the injection device, in a manner similar to that described hereinabove for the embodiments of Figures 29 to 31. The embodiment benefits from the aforementioned advantages, i.e. that the cartridge or ampoule is fillable with liquids in sterile conditions adapted to large volumes and in doses adapted to the specified uses without influencing the construction of the other portions of the container. The container portion 8"" containing the liquid to be injected, which forms an ampoule, may subsequently be assembled in the container portion 9"' containing the compressible substance. In this embodiment, the nozzle portion 11"' provided with the orifice 16' is integrally formed with the wall of the container portion 8"" containing the liquid to be injected.

In the embodiment of Fig. 34, a pressure peak (pressure shock) in the initial phase of injection is obtained by acceleration of the compressible liquid in the tube 76.

In the embodiment of Fig. 35, a pressure peak (pressure shock) in the initial phase of injection is obtained by rapid expansion of the compressible liquid 7, followed by expansion of dissolved liquid gas undergoing a phase change.

The liquid compressible substance may be a polysiloxane oil that is for example compressed to a lesser degree than in previously described embodiments, but in which a liquefied gas is dissolved (such as carbon dioxide, and nitrogen oxides).

During actuation, the pressure transmitting member displaces rapidly due to decompression of the liquid substance 7. Once the pressure reaches the pressure of "liquid-gas" phase-change of the dissolved or liquefied gas in the compressible substance, the gas takes over and continues its expansion while propulsing the liquid to be injected at the phase-change pressure. The liquid-gas phase change of carbon dioxide occurs at about 70 bars at ambient temperature.

In this embodiment, the volume of compressible liquid 7 may be roughly the same as the volume of the liquid to be injected 2. The volume of liquefied gas may occupy one tenth the volume of compressible liquid 7. During expansion of the liquefied gas in gas bubbles 77, the pressure remains constant during flow of the liquid to be injected 2 through the nozzle orifice.

The pressure peak may be 5 to 20 times higher than the average injection pressure. This pressure peak enables the epidermis or corium to be easily pierced, thereby guaranteeing complete injection of the liquid product.

Another embodiment according to Fig. 35 comprises a compressible liquid 7 including liquefied gas 77 provided in one or more capsules having a pressure transmitting member formed by a deformable wall.

In this embodiment, the compressible liquid 7 creates a pressure peak during decompression, and subsequently, the expansion of the compressed gas capsules produces a lesser pressure which is nevertheless sufficient to complete injection of the liquid 2.

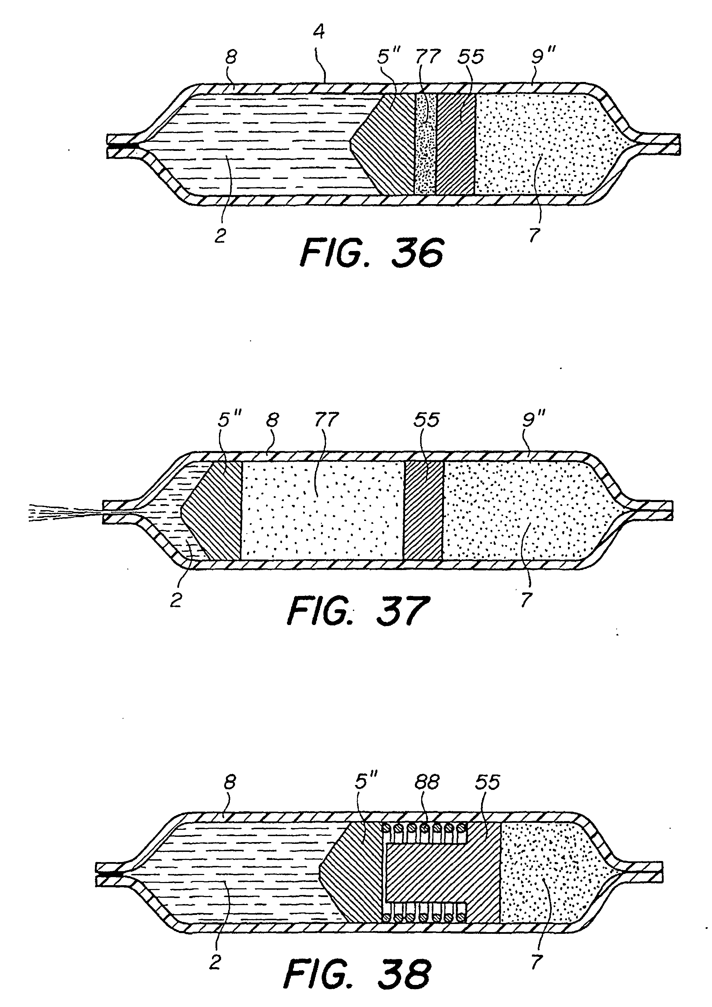

In the embodiment of Fig. 36, the compressible liquid 7 is separated from the liquefied or compressed gas 77 by a floating separating member such as a slidable piston 55 in the container 4. The compressible liquid 7 is compressed at about 500 bars (7% compressed), such that it displaces the piston by about 7% of its total displacement, the pressure of the liquid to be injected 2 is initially 500 bars which enables piercing of the epidermis or corium, and subsequently the liquefied or compressed gas takes over to complete evacuation of the liquid to be injected 2 at a pressure of around 70 bars for example, if carbon dioxide is used as the propulsion gas. Fig. 37 shows the position of the pistons 5" and 55 at the end of injection.

Fig. 38 shows a variant of Figures 36 and 37 in which the liquefied or compressed gas is replaced by a compressed spring 88 which provides the same effect as the compressed or liquefied gas.

In the latter embodiments, the pressure retaining means is represented by a paraffin plug in the nozzle orifice but other means may be used as described with respect to previous embodiments.

The compressible substance may comprise various organic oils or even water, although to the detriment of the volume necessary to obtain the same effect as with soft matter such as polysiloxanes. In the case of low viscosity fluids such as water, it would also be difficult to satisfy sealing requirements at the high pressures that are desired.

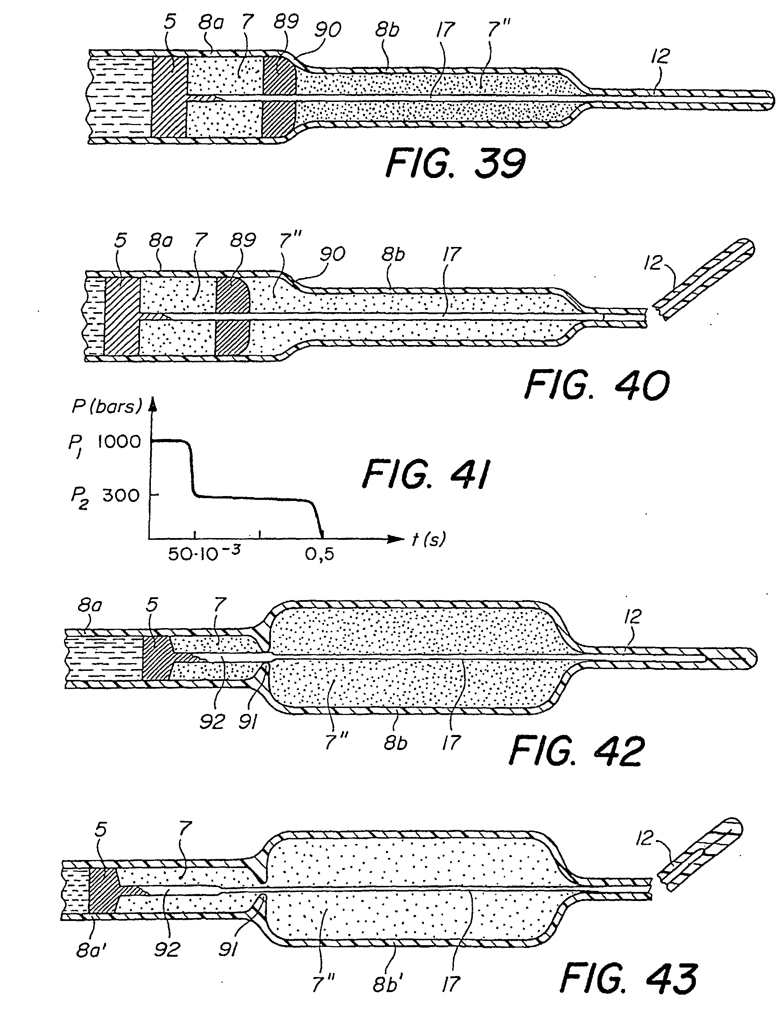

Figures 39 and 40 show another embodiment in which the container portion containing the liquid to be injected 2 comprises a first section 8a containing the compressible substance 7, and a second section 8b containing a compressible substance 7' that may either be the same of the compressible substance 7, for example soft matter as already described above, or a different substance that may be a liquid, solid or gaseous substance (as defined at ambient temperature and pressure). If the substance 7" is gaseous, it may be in the liquefied or gaseous state within the container depending on the pressure. The nozzle portion may take the various forms described for the above embodiments, although for simplicity the nozzle portion is shown integral with the container wall. The piston and retainer rod and means of liberating the piston may also comprise the various features of the previously described embodiments comprising a piston retained with a rod. The piston may also be free-floating by plugging the nozzle orifice in accordance with, for example, the various embodiments described above relating to the free-floating pistons.

A principal difference of this embodiment with respect to the previously described embodiments is the provision of a partition, in the form of a movable wall or piston 89 in the container portion comprising the compressible substances 7, 7" that is pushed against a stop, such as an abutment shoulder 90 prior to use. The abutment shoulder 90 is formed by an inward restriction of the outer wall of the container. The pressure of the compressible substance 7 in the container portion first section 8a is greater than in the container portion second section 8b, such that on actuation of the device, the piston 5 is initially driven by expansion of the first compressible substance 7 to give a peak injection pressure P1 as shown in Fig. 41. As P1 drops to pressure P2 of the second compressible substance 7" in the second section 8b, the movable partition 89 moves away from the abutment shoulder 90 allowing continued expansion of the second compressible substance 7" to complete injection at the lower pressure P2. This configuration has a similar propulsion effect to the embodiments of Figures 35 to 38, whereby a compressible liquid or solid at high pressure provides the initial peak injection pressure, for example to pierce the skin of a patient, followed by a lower pressure jet to complete injection of the liquid to be injected.

In certain applications, the above described two-stage injection jet pressure may be advantageous, particularly where the liquid to be injected should not penetrate too deep below the skin, as is generally the case for insulin injections, for example.

The embodiment of Figures 39 and 40, as for the embodiments of Figures 35 to 38, also enable the injection of very large doses in the case where the second compressible substance 7" is a compressed (or liquefied) gas.

In the embodiments of Figures 39 and 40, a further advantage is the particularly effective sealing of the second compressible substance 7" in the second section 8b, since the rear end of the container can be effectively sealed and the higher pressure of the compressible substance 7 in the first section 8a prevents leakage of the second compressible substance 7" towards the applicator end of the device. The compressible substance 7 may be selected from high molecular weight polysilixanes or a solid, such as vulcanised rubber that can be compressed under very high pressures in the range of 1000 to 3000 bars without escaping past the sealing joint between the piston and the inside of the container wall. This effect could be achieved with a very small quantity of compressible solid or liquid substance 7 in the first section 8a for implementation in designs where the potential energy is primarily in the form of compressed gas (as defined at ambient temperature and pressure) and would thus be an improvement with respect to conventional gas propulsion systems which are difficult to seal effectively.

The embodiments shown in Figures 42 and 43 have a similar functioning principle to the embodiments of Figures 39 and 40 in that there is a first section 8a' of a container portion for the compressible substance and a second section 8b' in which compressible substances 7 and 7" respectively, are stored at pressures P1 and P2 respectively. A main difference of this embodiment with the previously described embodiment is the fact that the two sections 8a', 8b' are separated by a reduced section passage 91 that is unplugged after an initial injection phase by displacement of the piston 5 which is provided with a plug portion 92 of larger diameter than the remaining portion of the rod 17 extending to the rear end of the device. The unplugging of the reduced section passage 91 causes the pressure to drop to P2 for the subsequent low pressure injection phase.

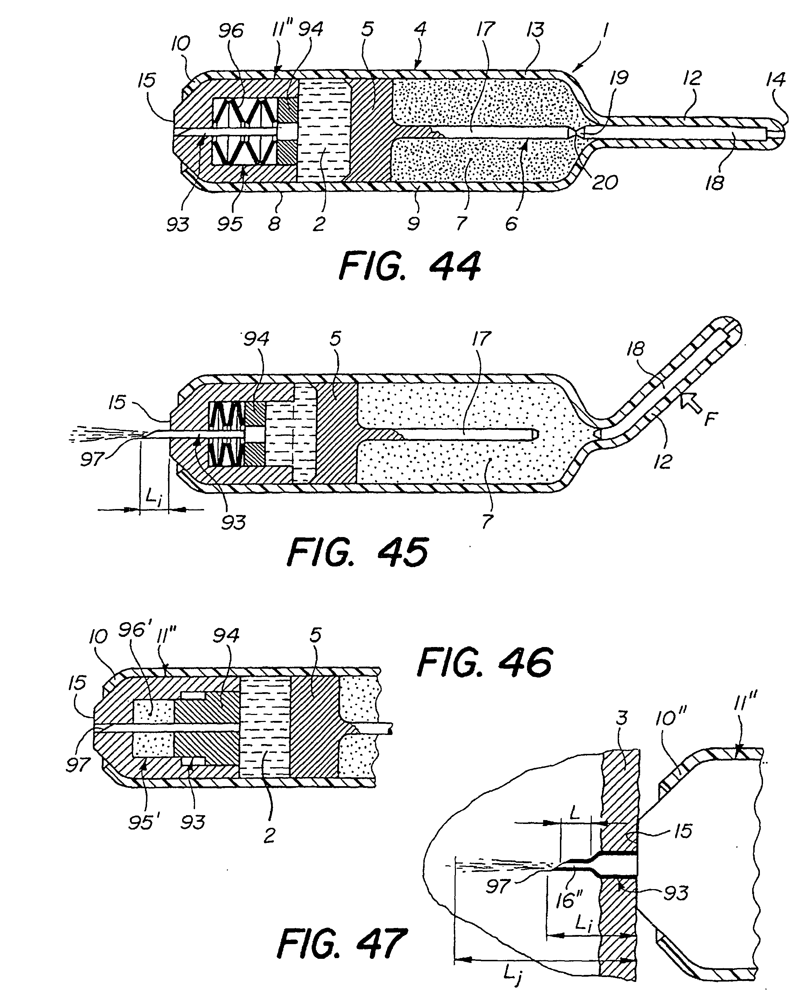

Referring to Figures 44 to 47, an embodiment comprising a propulsion system that may be similar to propulsion systems described above is shown, the main difference of this embodiment with respect to others residing in the design of the nozzle portion 11". The nozzle portion 11" of this embodiment is provided with a skin piercing member 93 comprising a needle that, during use, projects by a small amount Lᵢ corresponding roughly to the thickness of the epidermis for example to pierce through or to fragilise the skin of a patient. The skin piercing facilitates subcutaneous injection of the liquid to be injected. Intracutaneous injection is also possible if the needle (length Lᵢ) is correspondingly short, the injection pressure fairly low, and a spray is produced rather than a jet. The latter can be achieved, inter alia, by shortening the length L of the nozzle orifice 16".

The embodiments of Figures 44 to 47 can therefore be provided with a propulsion system generating lower pressure than required for a needleless injection device, at least as concerns the initial injection pressure required to pierce the skin. For reducing the risk of disease transmission that conventional needle syringes are subject to, the skin piercing member 93 comprises a piston or movable member 94 mounted against elastic buffer means 95, 95' that may either comprise mechanical spring elements, such as cup springs 96 or a compressible substance or member 96' as represented in Fig. 46, that maintains the piercing end 97 of the needle behind the application face 15 of the nozzle portion 11". During use, the pressure in the liquid to be injected 2 which is applied against the skin member piston 94, displaces the needle tip 97 beyond the application face 15. At the end of injection, the pressure drops below the spring force of the elastic buffer 95, 95' which retracts the needle behind the application face 15 as illustrated in Fig. 46. The needle 93 may integrally comprise the nozzle with orifice 16" adapted to control the quality of the jet as discussed with respect to the previous embodiments.

The nozzle portion 11" may also be attached to a membrane or other flexible container comprising the liquid to be injected and thus form a separate ampoule or cartridge mountable in or to the propulsion system in a similar manner to previously described embodiments.

It may be noted that with the advantageous propulsion system according to the invention, the needle may be significantly finer than the needles of conventional syringes. In addition, considering the short penetration length and short injection times which may be less than a second in view of the high pressure, there is a significantly increased comfort of use for patients with respect to conventional syringes. Furthermore, the combination of needle piercing depth and pressure of the propulsion system can be varied to accurately control the depth of the liquid injected, depending on the medical requirements. The rectractable needle eliminates the risk of disease transmission by piercing the skin of a person after use. A device with a non-movable skin-piercing member having a needle tip projecting permanently beyond the application end could however also be provided and would also benefit from the various advantageous aspects of a propulsion system according to this invention.

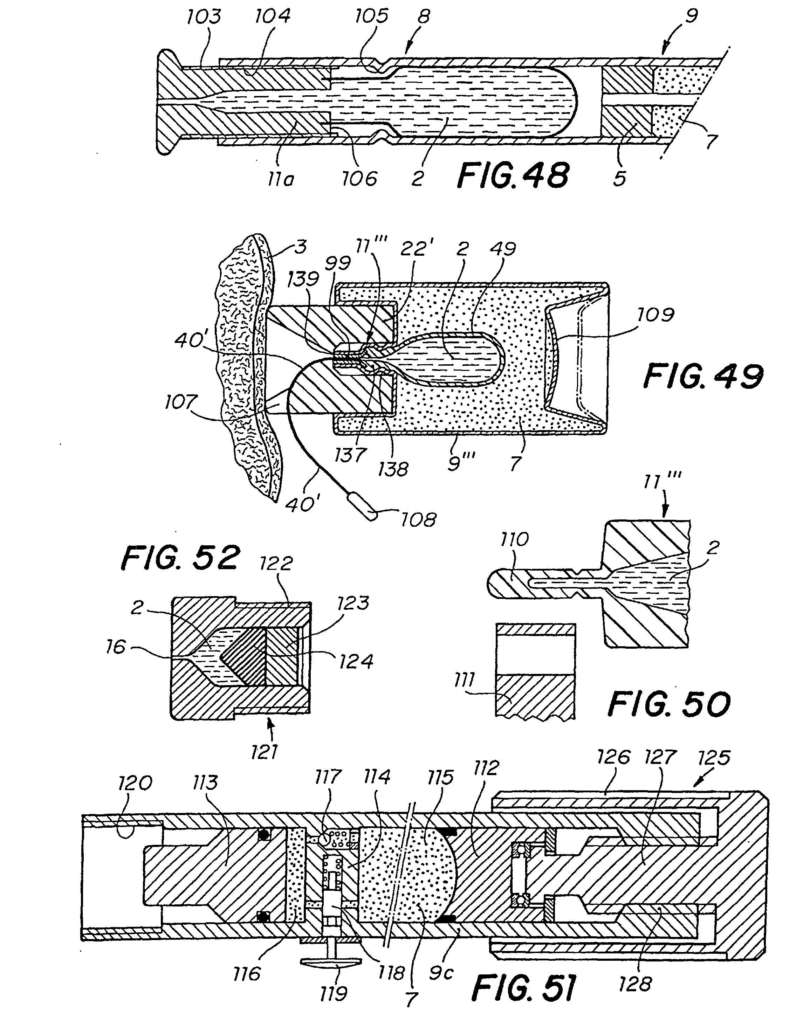

Referring to Fig. 48, an injection device with a propulsion system that may have the features of any one of the embodiments of Figures 1-16, 19-23, or 39-46, is provided with dosage adjustment means to vary the dosage of liquid to be injected. The dosage adjustment means comprises a nozzle portion 11 a having an outer threaded section 103 engaging in an inner threaded section 104 of the container portion 8 such that by turning the nozzle portion relative to the container portion 8, the nozzle portion is axially displaced towards, or away from the piston 5. The dosage adjustment means further comprises a stop 105 that defines the end position of the piston 54 during use. For injection of the maximum dosage, the nozzle portion is retreated until the rear end 106 thereof abuts the stop 105. For partial dosage, the nozzle portion is advanced, whereby the quantity of uninjected liquid 2 corresponds roughly to the container portion volume between the stop 105 and nozzle portion rear end 106.

Referring to figure 49, an injection device is shown comprising a container portion 9"' containing the compressible substance 7, and a deformable membrane 49 containing the liquid to be injected 2 within the container portion 9'''. The membrane 49 is attached to a nozzle portion 11''' that is mounted in the applicator end of the container portion 9''' and held therein by crimping in a collar portion 138. The nozzle orifice is blocked by a plug in the form of a wire 40' inserted through the applicator end of the nozzle portion. A support 22' for application of the device against the skin of a patient is provided with a groove 107 around which the wire 40' is guided. The wire extends to a handle 108 in order for the user to pull the wire out of the nozzle orifice to activate the device. In order to provide a good seal between the wire and the orifice, the nozzle portion tip 139 may be provided with a soft metal insert 99 as described in relation to embodiments of figures 18A and 18B.

The nozzle portion comprises a plastic insert 137 integrally formed with the membrane 49 and crimped to the container portion by indents 138. The actual outlet orifice is not provided in the insert, but in the nozzle tip 139 formed with the outer wall of the container portion 9"'.

In the embodiment of figure 49, the compressible substance 7 may be put under pressure by deforming the outer metal wall thereof, for example at a rear end 109, just prior to use. The deformation may be effected for example by means of a hand held hydraulic or lever-arm press or other mechanical crushing device. The advantage of putting the injection device under pressure just prior to use, is that it reduces the sealing requirements and prolongs the shelf life of the product.

The device of figure 49 may also have a propulsion system provided with a piston rather than a deformable membrane separating the liquid to be injected and compressible substance as described in relation to previous embodiments.

Referring to figure 50, a detailed partial view of a nozzle portion is shown comprising a breakable plug 110 that can be broken off by a ram 111 in order to actuate the device. The plug of figure 50 can be implemented in embodiments described above where the liquid to be injected is under pressure and the device is actuated by releasing or removing a plug.

Referring to figures 51 and 52, an embodiment of an injection device that is rechargeable is shown. The injection device comprises a container portion 9c, in which the compressible substance 7 is contained, a piston 112 closing a rear end and a piston 113 closing a front end of the container portion 9c. A separating wall 114 is provided inside the container portion 9c between the rear piston 112 and front piston 113. A large volume chamber 115 is formed between separating wall and the rear piston and a small volume chamber 116 is formed between the separating wall and the front piston. The separating wall is provided with a return valve 117 to allow compressible substance 7 from the front chamber 116 to flow into the rear chamber 115, whereby flow in the opposite direction is prevented. An actuation valve 118 is provided to allow the compressible substance to flow from the real chamber 115 to the front chamber 116 upon actuation of the valve, for example when the user presses a button 119 thereof.

The front end of the container portion 9c is provided with a threaded portion 120 for releasably mounting a capsule 121 containing the liquid to be injected, the capsule being provided with a complementary threaded portion 122. Other releasable fixing means could however be provided, such as a bayonet type connection or releasable spring latches. A rear end of the capsule is sealingly closed by a piston 123 that is driven by the propulsion unit piston 113 on actuation of the device thereby propulsing the liquid 2 through the nozzle orifice 16. The capsule piston 123 may be provided at it's front end with a cone shaped elastic member 124 in order to ensure that substantially all the liquid to be injected is propulsed out of the capsule.

A pressure generating mechanism 125 is mounted over the rear end of a container portion 9c and comprises a grip portion 126 and a ram portion 127 in the form of a threaded bolt engaging a complementary threaded portion 128 of the container portion 9c. As the mechanism 125 is screwed and the ram portion 127 is threaded into the container portion 9c, the piston 112 is displaced and compresses the compressible substance 7. The amount of turns applied to the grip 126 determines the pressure of the compressible substance 7 which can thus be adjusted according to the application. To actuate the device, the user opens the valve 118 by depressing the button 119 such that the compressible substance in the rear chamber 115 flows to the front chamber 116 and drives the piston 113 which drives the capsule piston 123. After use, the capsule 121 is removed from the propulsion unit and the pressure generating element 125 of the proportion unit is unwound to a position in which the compressible substance 7, when fully contained within with the rear chamber 115, is not under pressure. A new capsule 121 may then be fitted into the front end of the container portion 9c thereby pushing the propulsion unit front piston 113 back to the separating wall 114, the compressible substance 7 flowing from the front chamber 116 to the rear chamber 115 through the return valve 117. It is advantageous in this embodiment to have a compressible substance of low viscosity, such as a low molecular weight polysiloxane, such that the flow resistance through the valves 117 respectively 114 is low.

It is to be noted that the sealing requirements are less stringent for this embodiment than the embodiments that are supplied under pressure , in view of the short time between pressurising the compressible substance and injection.

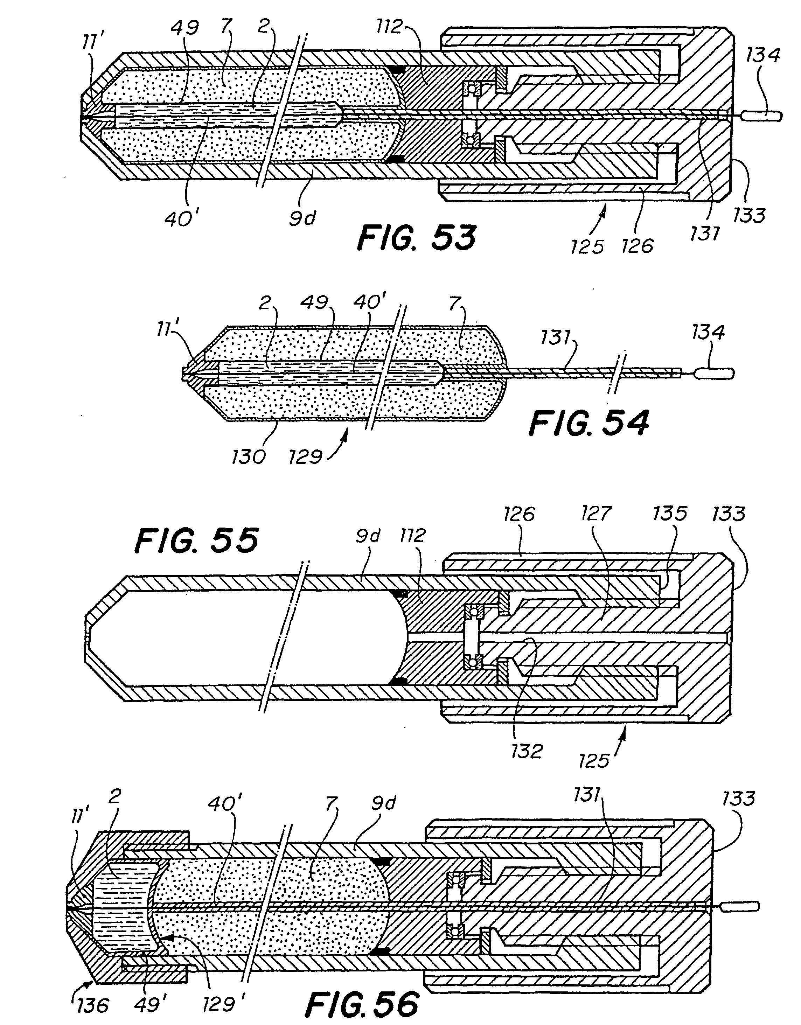

Referring to figures 53 and 54, another embodiment of an injection device is shown with a pressure generating mechanism which may be similar to the one described in relation to figure 51, mounted to a reusable container portion 9d for receiving a capsule 129 comprising the liquid to be injected 2 in a flexible membrane 49 surrounded (at least partially) by the compressible substance 7 in a membrane 130. If the compressible substance is silicon rubber or other compressible solid rather than a liquid polysiloxane, the membrane 130 is not necessary. The capsule further comprises a nozzle portion 11' with an outlet orifice blocked by a plug in the form of a high tensile strength wire 40'. The wire extends rearwardly through the membrane 49 into a long tail portion 131. The tail portion is received in a central passage 132 in the pressure generating mechanism extending through to the rear end 133 thereof such that the end 134 of the tail portion is accessible. The tail portion 131 may for example be made of plastic surrounding or encapsulating the wire 40'. As the wire is very fine, for example around 50 µm diameter, the frictional force retaining it is quite low and very easily overcome by a user pulling on the end 134 to actuate the device by liberating the nozzle orifice when the compressible substance is under operational pressure.

The container portion 9d can be made in two separable sections (not represented), or have a removable front end cap (similar to the embodiment of Fig. 56) in order to mount the capsule 129 therein. To apply pressure, the pressure generating mechanism is screwed inwardly after assembly of a new capsule.

Referring to figure 56, a variant of the embodiment of figure 53 is shown, in which the compressible substance 7 is mounted and remains in the container portion 9d' whereas the single-use capsule 129' is removably inserted in the front end of the device which is provided with a removable cap 136 that is screwed or assembled by other means to the container portion 9d'

The capsule 129' is provided with a wire 40' plugging the orifice of the nozzle 11' and extended in a tail portion 131 beyond a rear end 133 of the injection device in a similar manner to the embodiment of figure 53.

The capsule or ampoule membrane 49' is made, for example, of a plastic material, coated as appropriate for the pharmaceutical products contained therein. The nozzle portion 11' may have the features of above described nozzle portions, for example it may be provided with a metal nozzle tip embedded in a plastic body, the tip being provided with an outlet orifice formed by a ductile insert sealingly closed around the wire plug.

## Claims

1. A propulsion system suitable for a single-use or a multi-use injection device, said propulsion system comprising a container, a source of potential energy for propelling a fluid with sufficient pressure through an orifice to create a jet enabling subcutaneous or intracutaneous delivery of the fluid, the source of potential energy primarily in the form of a compressible substance under pressure within the container, whereby said potential energy is substantially compression energy of said substance, wherein said substance is a liquid, solid, or other non-gaseous substance as defined at ambient temperature and pressure.

2. Propulsion system according to claim 1, wherein the compressible substance has a volumetric compressibility (dV/V) at said pressure within the container greater than 1.2 times the volumetric compressibility of water.

3. Propulsion system according to any one of the preceding claims, wherein the compressible substance is put under pressure in the container by reducing the volume thereof after being filled with said compressible substance.

4. Propulsion system according to any one of the preceding claims, wherein the compressible substance is a visco-elastic liquid or soft matter.

5. Propulsion system according to the preceding claim, wherein the compressible substance belongs to the family of polysiloxanes.

6. Propulsion system according to any one of the preceding claims, wherein the device comprises a liquefied gas dissolved in or mixed with the compressible substance.

7. Propulsion system according to any one of claims 1 to 3, wherein the compressible substance is an elastic solid.

8. Propulsion system according to the preceding claim, wherein the solid is vulcanised silicon rubber.

9. Propulsion system according to any one of the preceding claims, wherein the pressure of the compressible substance in the container prior to use exceeds 200 bars.

10. Propulsion system according to anyone of the preceding claims, wherein the volume of compressible substance is reduced by a permanent deformation of a wall of the container.

11. Propulsion system according to any one of the preceding claims 1-9 wherein the volume of compressible substance is reduced by a pressure generating mechanism (125) of the device displacing a piston (112).

12. Propulsion system according to any one of the preceding claims 1-10, further comprising a movable or breakable separating or pressure transmitting member enclosing the compressible substance in the container, the separating or pressure transmitting member being adapted to be released or broken to enable the compressible substance to transmit pressure to said fluid to be injected.

13. Propulsion system according to claim 12, wherein said separating or pressure transmitting member is in the form of a piston maintained in position prior to use by retaining means.

14. Propulsion system according to claim 13, wherein the piston (5') comprises a first portion (36) subject to the pressure of the compressible substance (8), and a second portion (37) of smaller cross section than the first portion for applying a higher pressure than the pressure in the compressible substance on the fluid to be injected.

15. Propulsion system according to any one of the preceding claims, wherein the propulsion system forms a unit in which the compressible substance is under pressure, the unit being assemblable to an ampoule or capsule containing the fluid to be injected.

16. Propulsion system according to claim 12, wherein the piston (5", 5''') is mounted substantially floatably in the container

17. Propulsion system according to claim 12, wherein the separating or pressure transmitting member is a deformable wall (49, 49', 49", 49"").

18. Propulsion system according to any one of claims 1-11, further comprising retaining means comprising a plug (40, 40', 45, 47, 47') for maintaining the pressure of the compressible substance in the container prior to use by closing an orifice or a passage (16, 44, 44', 44", 44"').

19. Propulsion system according to the preceding claim, wherein the plug (40, 40', 47, 47') is a mechanical plug that may be displaced to liberate said passage or orifice.

20. Propulsion system according to claim 18, wherein the plug (47') is attached to a movable wall or piston (54) arranged in a container portion (9", 9"") containing the compressible substance such that, prior to use, a small amount of the compressible substance is positioned in a rear portion (60') of the container so as to maintain the piston in a position where the plug (47') blocks the passage (44, 44"').

21. Propulsion system according to the preceding claim, further comprising means to open the rear portion (60') for reducing pressure in this portion and causing displacement of the piston (44) and the plug (47') towards the rear.

22. A propulsion system suitable for a single use injection device, said propulsion system comprising a container, a source of potential energy for propelling a fluid with sufficient pressure through an orifice to create a jet enabling subcutaneous or intracutaneous delivery of the fluid, wherein the source of potential energy comprises a first compressible substance (7, 7') at a first pressure P1 within the container and at least a second compressible substance (7", 77) at a second pressure P2 lower than P1, whereby said potential energy is substantially compression energy of said substances, said first substance being a liquid, solid, or other non-gaseous substance as defined at ambient temperature and pressure.

23. Propulsion system according to the preceding claim, wherein the first compressible substance (7) has the composition and properties of the compressible substance as set forth in any one of claims 2, 3 and 6-10.

24. Propulsion system according to claim 22 or 23, wherein the first compressible substance (7) is enclosed in a first section (8a) of the container by a movable or breakable separating or pressure transmitting member adapted to be released or broken to enable the compressible substances to transmit pressure to said fluid to be injected.

25. Propulsion system according to the preceding claim, wherein the separating or pressure transmitting member has the features of the separating or pressure transmitting member as set forth in any one of claims 13 to 16.

26. Propulsion system according to any one of claims 22-25, further comprising a movable partition (89) separating a first section (8a) of the container comprising the first compressible substance from a second section (8b) of the container comprising the second compressible substance.

27. Propulsion system according to any one of claims 22-25, wherein a first section (8a) of the container comprising the first compressible substance is separated from a second section (8b) of the container comprising the second compressible substance by a reduced section passage (91) blocked by a plug portion (92) prior to use.

28. Propulsion system according to any one of claims 22-27, wherein the second compressible substance is a liquid or solid substance similar to the first compressible substance.

29. A single-use hypodermic injection device for subcutaneous or intracutaneous administration of a fluid product to be injected, such as a medicament, a vaccine or another pharmaceutical composition, comprising a propulsion system according to any one of the preceding claims, a fluid product to be injected and a nozzle portion having an orifice.

30. Device according to the preceding claim, wherein the fluid product to be injected (2) is contained in a separate ampoule or capsule or rigid cartridge, for mounting in or to the propulsion system.

31. Device according to claim 30, wherein the ampoule comprises a flexible or deformable wall fixed to the nozzle portion to contain the fluid to be injected therein.

32. Device according to claim 29, wherein the device comprises a slidable second free piston (55) separating a liquefied or compressed gas from the compressible substance (7).

33. Device according to claim 32, wherein the device comprises a compressed spring (88) instead of liquefied or compressed gas.

34. Device according to claim 29, wherein the container portion containing the liquid to be injected comprises a breakable partition such as a tube (76) that may be broken to actuate the device.

35. Device according to claim 29, wherein a plug is arranged in the nozzle portion (11, 11').

36. Device according to claim 29, wherein a plug of the propulsion system is arranged such that it blocks a passage (44, 44', 44", 44"') interconnecting a container portion containing the liquid to be injected and a container portion containing the compressible substance.

37. Device according to claim 35 wherein the wire is crimped in a ductile insert (99) of the nozzle portion, and defines the orifice diameter.

## Patentansprüche

1. Für eine Injektionsvorrichtung zum einmaligen oder mehrmaligen Gebrauch geeignetes Antriebssystem, das einen Behälter und eine Potentialenergiequelle enthält, um ein Fluid mit genügendem Druck durch eine Öffnung zu treiben und einen Strahl zu erzeugen, der eine subkutane oder intrakutane Abgabe des Fluids ermöglicht, wobei die Potentialenergiequelle primär in Gestalt einer verdichtbaren Substanz unter Druck innerhalb des Behälters vorliegt, diese Potentialenergie im Wesentlichen Verdichtungsenergie dieser Substanz ist und diese Substanz eine Flüssigkeit, ein Festkörper oder eine andere, nicht gasförmige Substanz ist, wie bei Umgebungsdruck und -temperatur definiert.

2. Antriebssystem nach Anspruch 1, worin die verdichtbare Substanz eine Volumenkompressibilität (dVN) bei dem genannten Druck im Behälter hat, die höher als 1,2-mal die Volumenkompressibilität von Wasser ist.

3. Antriebssystem nach einem der vorangehenden Ansprüche, worin die verdichtbare Substanz im Behälter unter Druck gesetzt wird, indem dessen Volumen verringert wird, nachdem er mit dieser verdichtbaren Substanz gefüllt worden ist.

4. Antriebssystem nach einem der vorangehenden Ansprüche, worin die verdichtbare Substanz eine viskoelastische Flüssigkeit oder weiche Materie ist.

5. Antriebssystem nach dem vorangehenden Anspruch, worin die verdichtbare Substanz zur Familie der Polysiloxane gehört.

6. Antriebssystem nach einem der vorangehenden Ansprüche, worin die Vorrichtung ein verflüssigtes Gas enthält, das in der verdichtbaren Substanz gelöst oder mit dieser vermischt ist.

7. Antriebssystem nach einem der Ansprüche 1 bis 3, worin die verdichtbare Substanz ein elastischer Festkörper ist.

8. Antriebssystem nach dem vorangehenden Anspruch, worin der Festkörper vulkanisierter Silikonkautschuk ist.

9. Antriebssystem nach einem der vorangehenden Ansprüche, worin der Druck der verdichtbaren Substanz im Behälter vor der Verwendung 200 bar übersteigt.

10. Antriebssystem nach einem der vorangehenden Ansprüche, worin das Volumen der verdichtbaren Substanz durch eine dauernde Verformung einer Behälterwandung verringert wird.

11. Antriebssystem nach einem der vorangehenden Ansprüche 1 bis 9, worin das Volumen der verdichtbaren Substanz durch einen druckerzeugenden Mechanismus (125) der Vorrichtung unter Verschiebung eines Kolbens (112) verringert wird.

12. Antriebssystem nach einem der vorangehenden Ansprüche 1 bis 10, weiter ein bewegbares oder brechbares Trennglied bzw. Druckübertragungsglied enthaltend, das die verdichtbare Substanz im Behälter umschliesst, wobei das Trenn- bzw. Druckübertragungsglied gelöst bzw. zerbrochen werden kann, um die verdichtbare Substanz in die Lage zu versetzen, Druck auf dieses zu injizierende Fluid zu übertragen.

13. Antriebssystem nach Anspruch 12, worin dieses Trenn- bzw. Druckübertragungsglied in Gestalt eines Kolbens vorliegt, der vor der Verwendung durch Rückhalteorgane an seinem Ort gehalten wird.

14. Antriebssystem nach Anspruch 13, worin der Kolben (5') einen ersten Abschnitt (36) aufweist, der dem Druck der verdichtbaren Substanz (8) ausgesetzt ist, sowie einen zweiten Abschnitt (37) mit einem kleineren Querschnitt als dem des ersten Abschnitts, um einen Druck auf das zu injizierende Fluid auszuüben, der höher als der Druck in der verdichtbaren Substanz ist.

15. Antriebssystem nach einem der vorangehenden Ansprüche, worin das Antriebssystem eine Einheit bildet, in der die verdichtbare Substanz unter Druck steht und die Einheit an eine Ampulle oder Kapsel angefügt werden kann, die das zu injizierende Fluid enthält.

16. Antriebssystem nach Anspruch 12, worin der Kolben (5", 5''') im Wesentlichen schwebend in den Behälter eingebaut ist.

17. Antriebssystem nach Anspruch 12, worin das Trenn- bzw. Druckübertragungsglied eine verformbare Wandung (49, 49', 49", 49"') ist.

18. Antriebssystem nach einem der Ansprüche 1 bis 11, weiter Rückhalteorgange mit einem Pfropfen (40, 40', 45, 47, 47') enthaltend, um vor der Verwendung den Druck der verdichtbaren Substanz im Behälter durch Verschluss einer Öffnung bzw. eines Kanals (16, 44, 44', 44", 44"') aufrechtzuerhalten .

19. Antriebssystem nach dem vorangehenden Anspruch, worin der Pfropfen (40, 40', 47, 47') ein mechanischer Pfropfen ist, der verschoben werden kann, um diesen Kanal bzw. diese Öffnung freizugeben.

20. Antriebssystem nach Anspruch 18, worin der Pfropfen (47') so an eine bewegbare Wandung bzw. an einen bewegbaren Kolben (54) angefügt ist, die in einem Abschnitt des Behälters (9", 9"") angeordnet sind, der die verdichtbare Substanz enthält, dass vor der Verwendung eine kleine Menge der verdichtbaren Substanz in einem rückwärtigen Abschnitt (60') des Behälters vorliegt, um den Kolben in einer Lage zu halten, in der der Pfropfen (47') den Kanal (44, 44"') versperrt.

21. Antriebssystem nach dem vorangehenden Anspruch, weiter Mittel enthaltend, um den rückwärtigen Abschnitt (60') zu öffnen, damit der Druck in diesem Abschnitt verringert und eine Verschiebung des Kolbens (44) und des Pfropfens (47') nach hinten veranlasst wird.

22. Für eine Injektionsvorrichtung zum einmaligen Gebrauch geeignetes Antriebssystem, das einen Behälter und eine Potentialenergiequelle enthält, um ein Fluid mit genügendem Druck durch eine Öffnung zu treiben und einen Strahl zu erzeugen, der eine subkutane oder intrakutane Abgabe des Fluids ermöglicht, wobei die Potentialenergiequelle eine erste verdichtbare Substanz (7, 7') unter einem ersten Druck P1 innerhalb des Behälters sowie zumindest eine zweite verdichtbare Substanz (7", 77) unter einem zweiten Druck P2 umfasst, der geringer als P1 ist, diese Potentialenergie im Wesentlichen Verdichtungsenergie dieser Substanzen ist und die erste Substanz eine Flüssigkeit, ein Festkörper oder eine andere, nicht gasförmige Substanz ist, wie bei Umgebungsdruck und -temperatur definiert.

23. Antriebssystem nach dem vorangehenden Anspruch, worin die erste verdichtbare Substanz (7) die Zusammensetzung und Eigenschaften der verdichtbaren Substanz hat, wie sie in einem beliebigen der Ansprüche 2, 3 und 6 bis 10 dargelegt sind.

24. Antriebssystem nach Anspruch 22 oder 23, worin die erste verdichtbare Substanz (7) in einem ersten Abschnitt (8a) des Behälters durch ein bewegbares oder brechbares Trennglied bzw. Druckübertragungsglied eingeschlossen wird, das gelöst bzw. zerbrochen werden kann, um die verdichtbare Substanz in die Lage zu versetzen, Druck auf dieses zu injizierende Fluid zu übertragen.

25. Antriebssystem nach dem vorangehenden Anspruch, worin das Trenn- bzw. Druckübertragungsglied die Merkmale des Trenn- bzw. Druckübertragungsgliedes hat, wie sie in einem der Ansprüche 13 bis 16 dargelegt sind.

26. Antriebssystem nach einem der Ansprüche 22 bis 25, weiter eine bewegbare Trennwand (89) aufweisend, die einen ersten Abschnitt (8a) des Behälters, der die erste verdichtbare Substanz enthält, von einem zweiten Abschnitt (8b) des Behälters trennt, der die zweite verdichtbare Substanz enthält.

27. Antriebssystem nach einem der Ansprüche 22 bis 25, worin ein erster Abschnitt (8a) des Behälters, der die erste verdichtbare Substanz enthält, von einem zweiten Abschnitt (8b) des Behälters, der die zweite verdichtbare Substanz enthält, durch einen Kanal (91) verringerten Querschnitts getrennt ist, der vor der Verwendung durch ein Pfropfenstück (92) versperrt wird.

28. Antriebssystem nach einem der Ansprüche 22 bis 27, worin die zweite verdichtbare Substanz eine der ersten verdichtbaren Substanz ähnliche Flüssigkeit bzw. feste Substanz ist.

29. Injektionsvorrichtung zum einmaligen Gebrauch für die subkutane oder intrakutane Verabreichung eines zu injizierenden fluiden Produkts wie eines Medikaments, eines Impfstoffes oder einer anderen pharmazeutischen Zusammensetzung, ein Antriebssystem nach einem der vorangehenden Ansprüche, ein zu injizierendes fluides Produkt sowie einen Düsenabschnitt mit einer Öffnung umfassend.

30. Vorrichtung nach dem vorangehenden Anspruch, worin das zu injizierende fluide Produkt (2) in einer getrennten Ampulle, Kaspel oder starren Patrone enthalten ist, die in oder an das Antriebssystem zu montieren ist.

31. Vorrichtung nach Anspruch 30, worin die Ampulle eine elastische oder verformbare, am Düsenabschnitt befestigte Wandung hat, die das zu injizierende Fluid darin hält.

32. Vorrichtung nach Anspruch 29, worin die Vorrichtung einen zweiten, frei gleitenden Kolben (55) enthält, der ein verflüssigtes oder verdichtetes Gas von der verdichtbaren Substanz (7) trennt.

33. Vorrichtung nach Anspruch 32, worin die Vorrichtung statt des verflüssigten oder verdichteten Gases eine zusammengedrückte Feder (88) enthält.

34. Vorrichtung nach Anspruch 29, worin der die zu injizierende Flüssigkeit enthaltende Behälterabschnitt eine brechbare Unterteilung wie zum Beispiel ein Rohr (76) enthält, das zerbrochen werden kann, um die Vorrichtung zu betätigen.

35. Vorrichtung nach Anspruch 29, worin ein Pfropfen im Düsenabschnitt (11, 11') angeordnet ist.

36. Vorrichtung nach Anspruch 29, worin ein Pfropfen des Antriebssystems so angeordnet ist, dass er einen Kanal (44, 44', 44", 44"') versperrt, der einen Behälterabschnitt, der die zu injizierende Flüssigkeit enthält, mit einem Behälterabschnitt verbindet, der die verdichtbare Substanz enthält.

37. Vorrichtung nach Anspruch 35, worin der Draht in einem biegsamen Einsatz (99) des Düsenabschnitts eingequetscht ist und den Öffnungsdurchmesser definiert.

## Revendications

1. Système propulseur convenant à un dispositif d'injection à usage unique ou à usage multiple, ledit système propulseur comprenant un récipient et une source d'énergie potentielle pour propulser un fluide par un orifice avec une pression suffisante pour créer un jet permettant une administration sous-cutanée ou intracutanée du fluide, et la source d'énergie potentielle se présentant, essentiellement, sous la forme d'une substance compressible sous pression à l'intérieur du récipient, où ladite énergie potentielle est, essentiellement, l'énergie de compression de ladite substance et où ladite substance est une substance liquide, une substance solide ou une autre substance qui est non gazeuse, lorsqu'elle se trouve à la température et à la pression ambiantes.

2. Système propulseur selon la revendication 1, dans lequel la substance compressible a une compressibilité volumique (dV / V) à ladite pression dans le récipient supérieure à 1,2 fois la compressibilité volumique de l'eau.

3. Système propulseur selon l'une quelconque des revendications précédentes, dans lequel la substance compressible est mise sous pression dans le récipient en diminuant son volume, après le remplissage du récipient avec ladite substance compressible.

4. Système propulseur selon l'une quelconque des revendications précédentes, dans lequel ladite substance compressible est un liquide viscoélastique ou un matériau mou.

5. Système propulseur selon la revendication précédente, dans lequel la substance compressible appartient à la famille des polysiloxanes.

6. Système propulseur selon l'une quelconque des revendications précédentes, dans lequel le dispositif comprend un gaz liquéfié dissous ou mélangé avec la substance compressible.

7. Système propulseur selon l'une quelconque des revendications 1 à 3, dans lequel la substance compressible est un solide élastique.

8. Système propulseur selon la revendication précédente, dans lequel le solide est un caoutchouc de silicone, vulcanisé.

9. Système propulseur selon l'une quelconque des revendications précédentes, dans lequel la pression de la substance compressible dans le récipient avant l'utilisation dépasse 200 bars.

10. Système propulseur selon l'une quelconque des revendications précédentes, dans lequel le volume de la substance compressible est diminué par une déformation permanente d'une paroi du récipient.

11. Système propulseur selon l'une quelconque des revendications précédentes
1 - 9, dans lequel le volume de la substance compressible est diminué par un mécanisme (125) générateur de pression du dispositif, déplaçant un piston (112).

12. Système propulseur selon l'une quelconque des revendications précédentes
1 - 10, comprenant, en outre, un élément séparateur ou capable de transmettre une pression, qui est déplaçable ou cassable et qui enferme la substance compressible dans le récipient, l'élément séparateur ou capable de transmettre une pression étant agencé pour être libéré ou cassé, afin de permettre à la substance compressible de transmettre une pression audit fluide à injecter.

13. Système propulseur selon la revendication 12, dans lequel ledit élément séparateur ou capable de transmettre une pression est réalisé sous la forme d'un piston maintenu en position avant l'utilisation, par des moyens de retenue.

14. Système propulseur selon la revendication 13, dans lequel le piston (5') comprend une première portion (36), exposée à la pression de la substance compressible (8), et une seconde portion (37) ayant une section transversale plus petite que la première portion, pour appliquer au fluide à injecter, une pression plus élevée que la pression dans la substance compressible.

15. Système propulseur selon l'une quelconque des revendications précédentes, où le système propulseur forme une unité dans laquelle la substance compressible est sous pression, l'unité pouvant être couplée avec une ampoule ou avec une capsule contenant le fluide à injecter.

16. Système propulseur selon la revendication 12, dans lequel le piston (5'', 5''') est monté d'une manière sensiblement flottante dans le récipient.

17. Système propulseur selon la revendication 12, dans lequel l'élément séparateur ou capable de transmettre une pression est une paroi déformable (49, 49', 49", 49"").

18. Système propulseur selon l'une quelconque des revendications 1 - 11, comprenant, en outre, des moyens de retenue comprenant un obturateur (40, 40', 45', 47, 47') pour conserver la pression de la substance compressible dans le récipient avant l'utilisation, par la fermeture d'un orifice ou d'un passage (16, 44, 44', 44", 44''').

19. Système propulseur selon la revendication précédente, dans lequel l'obturateur (40, 40', 47') est un obturateur mécanique qui peut être déplacé pour libérer ledit passage ou ledit orifice.

20. Système propulseur selon la revendication 18, dans lequel l'obturateur (47') est fixé à une paroi mobile ou à un piston (54) agencé dans une portion (9", 9"") du récipient contenant la substance compressible de manière à ce que, avant l'utilisation, une petite quantité de la substance compressible soit positionnée dans une portion arrière (60') du récipient, de manière à maintenir le piston dans une position où l'obturateur (47') ferme le passage (44, 44''').

21. Système propulseur selon la revendication précédente, comprenant, en outre, des moyens pour ouvrir la portion arrière (60'), afin de diminuer la pression dans cette portion et de provoquer le déplacement du piston (44) et de l'obturateur (47') vers l'arrière.

22. Système propulseur convenant à un dispositif d'injection à usage unique, ledit système propulseur comprenant un récipient et une source d'énergie potentielle pour propulser un fluide par un orifice avec une pression suffisante pour créer un jet permettant une administration sous-cutanée ou intracutanée du fluide, où la source d'énergie potentielle comprend une première substance compressible (7, 7') à une première pression P1 dans le récipient et au moins une seconde substance compressible (7", 77) à une seconde pression P2 inférieure à P1 et où ladite énergie potentielle est essentiellement l'énergie de compression desdites substances, ladite première substance étant une substance liquide, une substance solide ou une autre substance qui est non gazeuse lorsqu'elle se trouve à la température et à la pression ambiantes.

23. Système propulseur selon la revendication précédente, dans lequel la première substance compressible (7) a une composition et des propriétés de la substance compressible, telle qu'elle est définie dans une quelconque des revendications 2, 3 et 6 - 10.

24. Système propulseur selon la revendication 22 ou 23, dans lequel la première substance compressible (7) est enfermée dans une première section (8a) du récipient par un élément séparateur ou capable de transmettre une pression, qui est déplaçable ou cassable et qui est agencé pour être libéré ou cassé, afin de permettre aux substances compressibles de transmettre une pression audit fluide à injecter.

25. Système propulseur selon la revendication précédente, dans lequel l'élément séparateur ou capable de transmettre une pression a les traits caractéristiques de l'élément séparateur ou capable de transmettre une pression, tel qu'il est défini dans une quelconque des revendications 13 à 16.

26. Système propulseur selon l'une quelconque des revendications 22 - 25, comprenant, en outre, une paroi mobile (89) séparant une première section (8a) du récipient contenant la première substance compressible d'une seconde section (8b) du récipient contenant la seconde substance compressible.

27. Système propulseur selon l'une quelconque des revendications 22 - 25, dans lequel une première section (8a) du récipient, contenant la première substance compressible, est séparée d'une seconde section (8b) du récipient contenant la seconde substance compressible, par un passage à section diminuée (91) obstrué avant l'utilisation par une portion d'obturateur (92).

28. Système propulseur selon l'une quelconque des revendications 22 - 27, dans lequel la seconde substance compressible est une substance liquide ou une substance solide similaire à la première substance compressible.

29. Dispositif d'injection hypodermique à usage unique, pour une administration sous-cutanée ou intracutanée d'un produit fluide à injecter, tel qu'un médicament, un vaccin ou une autre composition pharmaceutique, comprenant un système propulseur selon l'une quelconque des revendications précédentes, un produit fluide à injecter et une portion de buse ayant un orifice.

30. Dispositif selon la revendication précédente, dans lequel le produit fluide à injecter (2) est contenu dans une ampoule, une capsule ou une cartouche rigide séparée, destinée à être montée sur ou dans le système propulseur.

31. Dispositif selon la revendication 30, dans lequel l'ampoule comprend une paroi flexible ou déformable, fixée à la portion de buse pour contenir le fluide à injecter.

32. Dispositif selon la revendication 29, où le dispositif comprend un second piston libre coulissant (55) séparant un gaz liquéfié ou comprimé de la substance compressible (7).

33. Dispositif selon la revendication 32, où le dispositif comprend un ressort comprimé (88) à la place du gaz liquéfié ou comprimé

34. Dispositif selon la revendication 29, dans lequel la portion de récipient contenant le liquide à injecter comprend une portion cassable, tel qu'un tube (76) qui peut être cassé pour actionner le dispositif.

35. Dispositif selon la revendication 29, dans lequel un obturateur est disposé dans la portion de buse (11, 11').

36. Dispositif selon la revendication 29, dans lequel un obturateur du système propulseur est disposé de manière à ce qu'il bloque un passage (44, 44', 44", 44"') reliant une portion du récipient contenant le liquide à injecter et une portion du récipient contenant la substance compressible.

37. Dispositif selon la revendication 35, dans lequel le fil métallique est serti dans une pièce insérée malléable (99) de la portion de buse et ce fil définit le diamètre de l'orifice.
